# EUROPEAN PATENT APPLICATION

(11) **EP 0 958 817 A1**
(43) Date of publication of application: **24.11.1999**
(21) Application number: 97913453.3
(22) Date of filing: 27.11.1997
(51) Int. Cl.: A61K 31/40, A61K 31/54, C07D 207/12, C07D 207/08, C07D 211/22, C07D 265/30, C07D 279/12

(54) **COMPOSITION CONTAINING DIARYLALKANE DERIVATIVE AS THE ACTIVE INGREDIENT FOR TREATING OR PREVENTING PANCREATITIS**

(30) Priority: 28.11.1996 JP 31774696
(71) Applicant: SANKYO COMPANY LIMITED, Chuo-ku, Tokyo 103-0023 (JP)
(72) Inventor: ASAI, Fumitoshi, Sankyo Company, Limited, Tokyo 140 (JP); FUJIMOTO, Koichi, Sankyo Company, Limited, Tokyo 140 (JP)
(74) Representative: Gibson, Christian John Robert
(86) International application number: JP9704323
(87) International publication number: WO9823271

(57) **Abstract**

A diarylalkane derivative represented by the following formula: 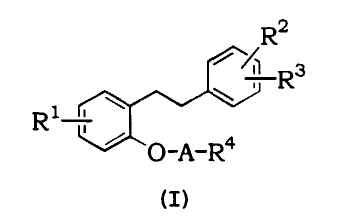 [wherein, R¹: a hydrogen atom, a halogen atom; R², R³: a hydrogen atom, a halogen atom, a C₁-C₄ alkoxy group; R⁴: a substituted or unsubstituted 5- or 6-membered cyclic amino group which may further contain an oxygen or sulfur atom; A: a C₁-C₄ alkylene group], or a pharmacologically acceptable salt thereof has excellent pancreatitis inhibitory activity and is therefore useful as a composition for the treatment or prevention of pancreatitis.

## Description

### [Technical Field]

The present invention relates to a composition for the treatment or prevention of pancreatitis which comprises a diarylalkane derivative or a pharmacologically acceptable salt thereof as an active ingredient; to usage of a diarylalkane derivative or a pharmacologically acceptable salt thereof to prepare an agent for the treatment or prevention of pancreatitis; or to a method for the treatment or prevention of pancreatitis which comprises administering to warm-blooded animals in need of such treatment or prevention an effective amount of a diarylalkane derivative or a pharmacologically acceptable salt thereof.

### [Background Art]

Diarylalkane derivatives are known to have antagonistic activity to serotonin 2 receptors and the like and to inhibit coagulation of blood platelets. They, therefore, are useful for the treatment or prevention of circulatory diseases [for example, J. Med. Chem., 35, 189 (1992), ibid., 33, 1818 (1990), Japanese Patent Application Kokai No. Hei 6-234736, Japanese Patent Application Kokai No. Hei 6-306025 and the like], however they have not been known to have inhibitory activity against pancreatitis.

### [Disclosure of the Invention]

The present inventors carried out extensive investigations for long years on the pharmacological activity of various diarylalkane derivatives. As a result, it has been found that diarylalkane derivatives have excellent pancreatitis inhibitory activity and are useful as a remedy or preventive for pancreatitis.

The present invention provides a composition for the treatment or prevention of pancreatitis which comprises a diarylalkane derivative or a pharmacologically acceptable salt thereof as an active ingredient; usage of a diarylalkane derivative or a pharmacologically acceptable salt thereof to prepare an agent for the treatment or prevention of pancreatitis; or a method for the treatment or prevention of pancreatitis, which comprises administering to warm-blooded animals in need of such treatment or prevention an effective amount of a diarylalkane derivative or a pharmacologically acceptable salt thereof.

The diarylalkane derivative, which is an active ingredient of the present invention, is represented by the following formula: 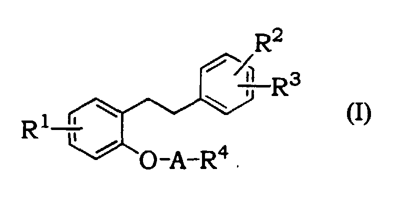 wherein:
R¹ represents a hydrogen atom or a halogen atom;
R² and R³ are the same or different and each independently represents a hydrogen atom, a halogen atom or a C₁-C₄ alkoxy group;
R⁴ represents a substituted or unsubstituted 5- or 6-membered cyclic amino group which may further contain an oxygen or sulfur atom (said substituent on a carbon atom is a hydroxy group or a C₁-C₂₀ aliphatic acyloxy group which may contain double bonds and said substituent on the nitrogen atom is a C₁-C₄ alkyl group); and
A represents a C₁-C₄ alkylene group.

Examples of the halogen atom of R¹, R² and R³ may be, for example, fluorine, chlorine, bromine or iodine atoms, of which the fluorine, chlorine or bromine atom is preferred and the fluorine or chlorine atom is more preferred.

Examples of the C₁-C₄ alkoxy group of R² and R³ may be, for example, methoxy, ethoxy, propoxy, isopropoxy, butoxy, isobutoxy, s-butoxy or t-butoxy groups, of which a methoxy or ethoxy group is preferred and a methoxy group is particularly preferred.

With respect to R⁴ examples of the 5- or 6-membered cyclic amino group which may further contain an oxygen or sulfur atom may be, for example, pyrrolidinyl, piperidyl, morpholinyl or thiomorpholinyl groups, of which a pyrrolidinyl, piperidyl or morpholinyl group is preferred, a pyrrolidinyl or piperidyl group is more preferred and a pyrrolidinyl group is the most preferred. In addition, the group A is preferably attached on a carbon atom of the cyclic amino group.

With respect to the C₁-C₂₀ aliphatic acyloxy group optionally containing double bonds which is a substituent of R⁴, examples of the C₁-C₂₀ aliphatic acyl moiety thereof may be, for example, formyl, acetyl, propionyl, butyryl, isobutyryl, valeryl, pivaloyl, hexanoyl, heptanoyl, octanoyl, nonanoyl, decanoyl, undecanoyl, lauroyl, myristoyl, palmitoyl, stearoyl, icosanoyl, acryloyl, methacryloyl, crotonoyl, oleoyl or linoleoyl groups, of which C₂-C₅ aliphatic acyl groups, C₈-C₁₈ aliphatic acyl groups, an acryloyl group, a crotonoyl group, an oleoyl group or a linoleoyl group are preferred, C₈-C₁₈ aliphatic acyl groups are more preferred, an octanoyl, decanoyl, lauroyl, myristoyl, palmitoyl or stearoyl group is still more preferred and a decanoyl or lauroyl group is the most preferred.

With respect to R⁴, examples of the C₁-C₄ alkyl substituent on the nitrogen atom may be, for example, methyl, ethyl, propyl, isopropyl, butyl, isobutyl, s-butyl or t-butyl groups, of which a methyl or ethyl group is preferred and a methyl group is particularly preferred.

Specific examples of the substituted or unsubstituted 5- or 6-membered cyclic amino group which may further contain an oxygen or sulfur atom may be, for example, pyrrolidinyl, methylpyrrolidinyl, ethylpyrrolidinyl, propylpyrrolidinyl, isopropylpyrrolidinyl, butylpyrrolidinyl, hydroxypyrrolidinyl, formyloxypyrrolidinyl, acetoxypyrrolidinyl, propionyloxypyrrolidinyl, butyryloxypyrrolidinyl, valeryloxypyrrolidinyl, pivaloyloxypyrrolidinyl, hexanoyloxypyrrolidinyl, heptanoyloxypyrrolidinyl, octanoyloxypyrrolidinyl, nonanoyloxypyrrolidinyl, decanoyloxypyrrolidinyl, undecanoyloxypyrrolidinyl, lauroyloxypyrrolidinyl, myristoyloxypyrrolidinyl, palmitoyloxypyrrolidinyl, stearoyloxypyrrolidinyl, icosanoyloxypyrrolidinyl, acryloyloxypyrrolidinyl, methacryloyloxypyrrolidinyl, crotonoyloxypyrrolidinyl, oleoyloxypyrrolidinyl, linolcoyloxypyrrolidinyl, 1-methyl-hydroxypyrrolidinyl, 1-methyl-formyloxypyrolidiyl, 1-methyl-acetoxypyrrolidinyl, 1-methyl-propionyloxypyrrolidinyl, 1-methyl-butyryloxypyrrolidinyl, 1-methyl-valeryloxypyrrolidinyl, 1-methyl-pivaloyloxypyrrolidinyl, 1-methyl-hexanoyloxypyrrolidinyl, 1-methyl-3,3-dimethylbutyryloxypyrrolidinyl, 1-methyl-heptanoyloxypyrrolidinyl, 1-methyl-octanoyloxypyrrolidinyl, 1-methyl-nonanoyloxypyrrolidinyl, 1-methyl-decanoyloxypyrrolidinyl, 1-methyl-undecanoyloxypyrrolidinyl, 1-methyl-lauroyloxypyrrolidinyl, 1-methyl-myristoyloxypyrrolidinyl, 1-methyl-palmitoyloxypyrrolidinyl, 1-methyl-stearoyloxypyrrolidinyl, 1-methyl-icosanoyloxypyrrolidinyl, 1-methyl-acryloyloxypyrrolidinyl, 1-methyl-methacryloyloxypyrrolidinyl, 1-methyl-crotonoyloxypyrrolidinyl, 1-methyl-oleoyloxypyrrolidinyl, 1-methyl-linoleoyloxypyrrolidinyl, 1-ethyl-hydroxypyrrolidinyl, 1-ethyl-acetoxypyrrolidinyl, 1-ethyl-propionyloxypyrrolidinyl, 1-ethyl-butyryloxypyrrolidinyl, 1-ethyl-valeryloxypyrrolidinyl, 1-ethyl-pivaloyloxypyrrolidinyl, 1-ethyl-octanoyloxypyrrolidinyl, 1-ethyl-nonanoyloxypyrrolidinyl, 1-ethyl-decanoyloxypyrrolidinyl, 1-ethyl-undecanoyloxypyrrolidinyl, 1-ethyl-lauroyloxypyrrolidinyl, 1-ethyl-myristoyloxypyrrolidinyl, 1-ethyl-palmitoyloxypyrrolidinyl, 1-ethyl-stearoyloxypyrrolidinyl, 1-ethyl-acryloyloxypyrrolidinyl, 1-ethyl-crotonoyloxypyrrolidinyl, 1-ethyl-linoleoyloxypyrrolidinyl, piperidyl, methylpiperidyl, ethylpiperidyl, propylpiperidyl, isopropylpiperidyl, butylpiperidyl, hydroxypiperidyl, acetoxypiperidyl, propionyloxypiperidyl, butyryloxypiperidyl, valeryloxypiperidyl, pivaloyloxypiperidyl, decanoyloxypiperidyl, lauroyloxypiperidyl, myristoyloxypiperidyl, palmitoyloxypiperidyl, stearoyloxypiperidyl, acryloyloxypiperidyl, linoleoyloxypiperidyl, 1-methyl-hydroxypiperidyl, 1-methyl-acetoxypiperidyl, 1-methyl-propionyloxypiperidyl, 1-methyl-butyryloxypiperidyl, 1-methyl-valeryloxypiperidyl, 1-methyl-pivaloyloxypiperidyl, 1-methyl-decanoyloxypiperidyl, 1-methyl-lauroyloxypiperidyl, 1-methyl-myristoyloxypiperidyl, 1-methyl-palmitoyloxypiperidyl, 1-methyl-stearoyloxypiperidyl, 1-methylacryloyloxypiperidyl, 1-methyl-linoleoyloxypiperidyl, 1-ethyl-hydroxypiperidyl, 1-ethyl-acetoxypiperidyl, 1-ethyl-propionyloxypiperidyl, 1-ethyl-butyryloxypiperidyl, 1-ethyl-valeryloxypiperidyl, 1-ethyl-pivaloyloxypiperidyl, 1-ethyl-decanoyloxypiperidyl, 1-ethyl-lauroyloxypiperidyl, 1-ethyl-myristoyloxypiperidyl, 1-ethyl-palmitoyloxypiperidyl, 1-ethyl-stearoyloxypiperidyl, 1-ethyl-acryloyloxypiperidyl, 1-ethyl-linoleoyloxypiperidyl, morpholinyl, 4-methylmorpholinyl, 4-ethylmorpholinyl, 4-propylmorpholinyl, 4-isoproylmorpholinyl, 4-butylmorpholinyl, thiomorpholinyl, 4-methylthiomorpholinyl, 4-ethylthiomorpholinyl, 4-propylthiomorpholinyl, 4-isopropylthiomorpholinyl or 4-butylthiomorpholinyl groups;
of which a pyrrolidinyl, methylpyrrolidinyl, ethylpyrrolidinyl, hydroxypyrrolidinyl, acetoxypyrrolidinyl, propionyloxypyrrolidinyl, valeryloxypyrrolidinyl, pivaloyloxypyrrolidinyl, octanoyloxypyrrolidinyl, decanoyloxypyrrolidinyl, undecanoyloxypyrrolidinyl, lauroyloxypyrrolidinyl, myristoyloxypyrrolidinyl, palmitoyloxypyrrolidinyl, stearoyloxypyrrolidinyl, acryloyloxypyrrolidinyl, linoleoyloxypyrrolidinyl, 1-methyl-hydroxypyrrolidinyl, 1-methylacetoxypyrolidinyl, 1-methyl-propionyloxypyrrolidinyl, 1-methylvaleryloxypyrrolidinyl, 1-methyl-pivaloyloxypyrrolidinyl, 1-methyl-octanoyloxypyrrolidinyl, 1-methyl-decanoyloxypyrrolidinyl, 1-methyl-undecanoyloxypyrrolidinyl, 1-methyl-lauroyloxypyrrolidinyl, 1-methyl-myristoyloxypyrrolidinyl, 1-methyl-palmitoyloxypyrrolidinyl, 1-methyl-stearoyloxypyrrolidinyl, 1-methyl-acryloyloxypyrrolidinyl, 1-methyl-linoleoyloxypyrrolidinyl, 1-ethyl-hydroxypyrrolidinyl, 1-ethyl-acetoxypyrrolidinyl, 1-ethyl-propionyloxypyrrolidinyl, 1-ethyl-valeryloxypyrrolidinyl, 1-ethyl-pivaloyloxypyrrolidinyl, 1-ethyl-decanoyloxypyrrolidinyl, 1-ethyl-lauroyloxypyrrolidinyl, 1-ethylmyristoyloxypyrrolidinyl, 1-ethyl-palmitoyloxypyrrolidinyl, 1-ethyl-stearoyloxypyrrolidinyl, 1-ethyl-linoleoyloxypyrrolidinyl, piperidyl, methylpiperidyl, ethylpiperidyl, hydroxypiperidyl, acetoxypiperidyl, propionyloxypiperidyl, valeryloxypiperidyl, pivaloyloxypiperidyl, decanoyloxypiperidyl, lauroyloxypiperidyl, myristoyloxypiperidyl, palmitoyloxypiperidyl, stearoyloxypiperidyl, acryloyloxypiperidyl, linoleoyloxypiperidyl, 1-methyl-hydroxypiperidyl, 1-methyl-acetoxypiperidyl, 1-methyl-propionyloxypiperidyl, 1-methyl-valeryloxypiperidyl, 1-methyl-pivaloyloxypiperidyl, 1-methyl-decanoyloxypiperidyl, 1-methyl-lauroyloxypiperidyl, 1-methyl-myristoyloxypiperidyl, 1-methyl-palmitoyloxypiperidyl, 1-methyl-stearoyloxypiperidyl, 1-methylacryloyloxypiperidyl, 1-methyl-linoleoyloxypiperidyl, 1-ethyl-hydroxypiperidyl, 1-ethyl-acetoxypiperidyl, 1-ethyl-propionyloxypiperidyl, 1-ethyl-valeryloxypiperidyl, 1-ethyl-pivaloyloxypiperidyl, 1-ethyl-decanoyloxypiperidyl, 1-ethyl-lauroyloxypiperidyl, 1-ethyl-myristoyloxypiperidyl, 1-ethyl-palmitoyloxypiperidyl, 1-ethyl-stearoyloxypiperidyl, 1-ethyl-linoleoyloxypiperidyl, morpholinyl, 4-methylmorpholinyl, 4-ethylmorpholinyl, thiomorpholinyl, 4-methylthiomorpholinyl or 4-ethylthiomorpholinyl group is preferred;
a 2-pyrrolidinyl, 3-pyrrolidinyl, 1-methyl-2-pyrrolidinyl, 1-methyl-3-pyrrolidinyl, 4-hydroxy-2-pyrrolidinyl, 4-acetoxy-2-pyrrolidinyl, 4-propionyloxy-2-pyrrolidinyl, 4-valeryloxy-2-pyrrolidinyl, 4-pivaloyloxy-2-pyrrolidinyl, 4-octanoyloxy-2-pyrrolidinyl, 4-decanoyloxy-2-pyrrolidinyl, 4-lauroyloxy-2-pyrrolidinyl, 4-myristoyloxy-2-pyrrolidinyl, 4-palmitoyloxy-2-pyrrolidinyl, 4-stearoyloxy-2-pyrrolidinyl, 1-methyl-4-hydroxy-2-pyrrolidinyl, 1-methyl-4-acetoxy-2-pyrrolidinyl, 1-methyl-4-propionyloxy-2-pyrrolidinyl, 1-methyl-4-valeryloxy-2-pyrrolidinyl, 1-methyl-4-pivaloyloxy-2-pyrrolidinyl, 1-methyl-4-octanoyloxy-2-pyrrolidinyl, 1-methyl-4-decanoyloxy-2-pyrrolidinyl, 1-methyl-4-lauroyloxy-2-pyrrolidinyl, 1-methyl-4-myristoyloxy-2-pyrrolidinyl, 1-methyl-4-palmitoyloxy-2-pyrrolidinyl, 1-methyl-4-stearoyloxy-2-pyrrolidinyl, 1-ethyl-4-hydroxy-2-pyrrolidinyl, 1-ethyl-4-acetoxy-2-pyrrolidinyl, 1-ethyl-4-decanoyloxy-2-pyrrolidinyl, 1-ethyl-4-lauroyloxy-2-pyrrolidinyl, 1-ethyl-4-myristoyloxy-2-pyrrolidinyl, 1-ethyl-4-palmitoyloxy-2-pyrrolidinyl, 1-ethyl-4-stearoyloxy-2-pyrrolidinyl, 2-piperidyl, 3-piperidyl, 4-piperidyl, 1-methyl-2-piperidyl, 1-methyl-3-piperidyl, 1-methyl-4-piperidyl, 4-hydroxy-2-piperidyl, 1-methyl-4-hydroxy-2-piperidyl, 2-morpholinyl, 3-morpholinyl, 4-methyl-2-morpholinyl, 4-methyl-3-morpholinyl, 4-ethyl-2-morpholinyl, 2-thiomorpholinyl or 4-methyl-2-thiomorpholinyl group is more preferred;
a 2-pyrrolidinyl, 3-pyrrolidinyl, 1-methyl-2-pyrrolidinyl, 4-hydroxy-2-pyrrolidinyl, 4-acetoxy-2-pyrrolidinyl, 4-pivaloyloxy-2-pyrrolidinyl, 4-octanoyloxy-2-pyrrolidinyl, 4-decanoyloxy-2-pyrrolidinyl, 4-lauroyloxy-2-pyrrolidinyl, 4-myristoyloxy-2-pyrrolidinyl, 4-palmitoyloxy-2-pyrrolidinyl, 4-stearoyloxy-2-pyrrolidinyl, 1-methyl-4-hydroxy-2-pyrrolidinyl, 1-methyl-4-acetoxy-2-pyrrolidinyl, 1-methyl-4-pivaloyloxy-2-pyrrolidinyl, 1-methyl-4-octanoyloxy-2-pyrrolidinyl, 1-methyl-4-decanoyloxy-2-pyrrolidinyl, 1-methyl-4-lauroyloxy-2-pyrrolidinyl, 1-methyl-4-myristoyloxy-2-pyrrolidinyl, 1-methyl-4-palmitoyloxy-2-pyrrolidinyl, 1-methyl-4-stearoyloxy-2-pyrrolidinyl, 2-piperidyl, 3-piperidyl, 4-piperidyl, 1-methyl-2-piperidyl, 1-methyl-3-piperidyl, 1-methyl-4-piperidyl, 2-morpholinyl, 4-methyl-2-morpholinyl or 2-thiomorpholinyl group is still more preferred;
a 2-pyrrolidinyl, 3-pyrrolidinyl, 1-methyl-2-pyrrolidinyl, 4-hydroxy-2-pyrrolidinyl, 4-octanoyloxy-2-pyrrolidinyl, 4-decanoyloxy-2-pyrrolidinyl, 4-lauroyloxy-2-pyrrolidinyl, 4-myristoyloxy-2-pyrrolidinyl, 4-palmitoyloxy-2-pyrrolidinyl, 4-stearoyloxy-2-pyrrolidinyl, 1-methyl-4-hydroxy-2-pyrrolidinyl, 1-methyl-4-octanoyloxy-2-pyrrolidinyl, 1-methyl-4-decanoyloxy-2-pyrrolidinyl, 1-methyl-4-lauroyloxy-2-pyrrolidinyl, 1-methyl-4-myristoyloxy-2-pyrrolidinyl, 1-methyl-4-palmitoyloxy-2-pyrrolidinyl, 1-methyl-4-stearoyloxy-2-pyrrolidinyl, 2-piperidyl, 3-piperidyl, 4-piperidyl, 1-methyl-2-piperidyl, 1-methyl-3-piperidyl, 1-methyl-4-piperidyl, 2-morpholinyl or 4-methyl-2-morpholinyl group is still much more preferred; and
a 2-pyrrolidinyl, 1-methyl-2-pyrrolidinyl, 4-hydroxy-2-pyrrolidinyl, 4-decanoyloxy-2-pyrrolidinyl, 4-lauroyloxy-2-pyrrolidinyl, 1-methyl-4-hydroxy-2-pyrrolidinyl, 1-methyl-4-decanoyloxy-2-pyrrolidinyl or 1-methyl-4-lauroyloxy-2-pyrrolidinyl group is the most preferred.

Examples of the C₁-C₄ alkylene group of A may be, for example, methylene, ethylene, propylene, trimethylene or tetramethylene groups, of which C₁-C₃ alkylene groups are preferred, a methylene or ethylene group is more preferred and an ethylene group is the most preferred.

The compound (I), which serves as an active ingredient of the present invention can be convened into the corresponding pharmacologically acceptable acid addition salt by treating with an acid according to a conventional method. Examples of the acid addition salt include salts of an inorganic acid such as hydrochloric acid, hydrobromic acid, sulfuric acid or phosphoric acid; salts of an organic acid such as acetic acid, benzoic acid, oxalic acid, maleic acid, fumaric acid, tartaric acid or citric acid; and salts of a sulfonic acid such as methanesulfonic acid, benzenesulfonic acid or p-toluenesulfonic acid; of which the salt of hydrochloric acid is preferred.

The compound (I) or pharmacologically acceptable salt thereof absorbs water when it is allowed to stand in the atomosphere, whereby they have adsorbed water to hold water or to form a hydrate. Such a derivative is also included in the present invention as an active ingredient.

If the compound (I) has an asymmetric carbon in the molecule thereof, there exist optical isomers and a mixture thereof in any proportion. These substances are also included in the present invention as an active ingredient.

Following compounds having the formula (I) are preferred.
(1) compounds wherein R¹ represents a hydrogen, fluorine or chlorine atom,
(2) compounds wherein R¹ represents a hydrogen or fluorine atom,
(3) compounds wherein R² and R³ are the same or different and each independently represents a hydrogen atom, fluorine atom, chlorine atom, methoxy group or ethoxy group,
(4) compounds wherein R² represents a hydrogen, fluorine or chlorine atom and R³ represents a methoxy group,
(5) compounds wherein R² represents a hydrogen or fluorine atom and R³ represents a methoxy group,
(6) compounds wherein R⁴ represents a, substituted or unsubstituted, pyrrolidinyl, piperidyl, morpholinyl or thiomorpholinyl group (said substituent on a carbon atom is a hydroxy, C₂-C₅ aliphatic acyloxy, C₈-C₁₈ aliphatic acyloxy, acryloyl, crotonoyl, oleoyl or linoleoyl group and said substituent on the nitrogen atom is a methyl or ethyl group),
(7) compounds wherein R⁴ represents a, substituted or unsubstituted, pyrrolidinyl, piperidyl or morpholinyl group (said substituent on the carbon atom is a hydroxy or C₈-C₁₈ aliphatic acyloxy group and said substituent on the nitrogen atom is a methyl group),
(8) compounds wherein R⁴ represents a 2-pyrrolidinyl, 3-pyrrolidinyl, 1-methyl-2-pyrrolidinyl, 4-hydroxy-2-pyrrolidinyl, 4-octanoyloxy-2-pyrrolidinyl, 4-decanoyloxy-2-pyrrolidinyl, 4-lauroyloxy-2-pyrrolidinyl, 4-myristoyloxy-2-pyrrolidinyl, 4-palmitoyloxy-2-pyrrolidinyl, 4-stearoyloxy-2-pyrrolidinyl, 1-methyl-4-hydroxy-2-pyrrolidinyl, 1-methyl-4-octanoyloxy-2-pyrrolidinyl, 1-methyl-4-decanoyloxy-2-pyrrolidinyl, 1-methyl-4-lauroyloxy-2-pyrrolidinyl, 1-methyl-4-myristoyloxy-2-pyrrolidinyl, 1-methyl-4-palmitoyloxy-2-pyrrolidinyl, 1-methyl-4-stearoyloxy-2-pyrrolidinyl, 2-piperidyl, 3-piperidyl, 4-piperidyl, 1-methyl-2-piperidyl, 1-methyl-3-piperidyl, 1-methyl-4-piperidyl, 2-morpholinyl or 4-methyl-2-morpholinyl group,
(9) compounds wherein R⁴ represents a 2-pyrrolidinyl, 1-methyl-2-pyrrolidinyl, 4-hydroxy-2-pyrrolidinyl, 4-decanoyloxy-2-pyrrolidinyl, 4-lauroyloxy-2-pyrrolidinyl, 1-methyl-4-hydroxy-2-pyrrolidinyl, 1-methyl-4-decanoyloxy-2-pyrrolidinyl or 1-methyl-4-lauroyloxy-2-pyrrolidinyl group,
(10) compounds wherein A represents a C₁-C₃ alkylene group,
(11) compounds wherein A represents a methylene or ethylene group, or
(12) compounds wherein A represents an ethylene group.
   In addition, compounds of combinations selected freely from respective groups of (1) - (2), (3) - (5), (6) - (9) and (10) - (12) are preferred. Examples are;
(13) compounds wherein R¹ represents a hydrogen, fluorine or chlorine atom,
   R² and R³ are the same or different and each independently represents a hydrogen atom, fluorine atom, chlorine atom, methoxy group or ethoxy group,
   R⁴ represents a, substituted or unsubstituted, pyrrolidinyl, piperidyl, morpholinyl or thiomorpholinyl group (said substituent on a carbon atom is a hydroxy group, C₂-C₅ aliphatic acyloxy group, C₈-C₁₈ aliphatic acyloxy group, acryloyl group, crotonoyl group, oleoyl group or linoleoyl group and said substituent on the nitrogen atom is a methyl or ethyl group), and
   A represents a C₁-C₃ alkylene group,
(14) compounds wherein R¹ represents a hydrogen or fluorine atom,
   R² represents a hydrogen atom, fluorine atom or chlorine atom, R³ represents a methoxy group,
   R⁴ represents a, substituted or unsubstituted, pyrrolidinyl, piperidyl or morpholinyl group (said substituent on a carbon atom is a hydroxy group or C₈-C₁₈ aliphatic acyloxy group and said substituent on the nitrogen atom is a methyl group), and
   A represents a methylene or ethylene group,
(15) compounds wherein R¹ represents a hydrogen or fluorine atom,
   R² represents a hydrogen atom, fluorine atom or chlorine atom, R³ represents a methoxy group,
   R⁴ represents a 2-pyrrolidinyl, 3-pyrrolidinyl, 1-methyl-2-pyrrolidinyl, 4-hydroxy-2-pyrrolidinyl, 4-octanoyloxy-2-pyrrolidinyl, 4-decanoyloxy-2-pyrrolidinyl, 4-lauroyloxy-2-pyrrolidinyl, 4-myristoyloxy-2-pyrrolidinyl, 4-palmitoyloxy-2-pyrrolidinyl, 4-stearoyloxy-2-pyrrolidinyl, 1-methyl-4-hydroxy-2-pyrrolidinyl, 1-methyl-4-octanoyloxy-2-pyrrolidinyl, 1-methyl-4-decanoyloxy-2-pyrrolidinyl, 1-methyl-4-lauroyloxy-2-pyrrolidinyl, 1-methyl-4-myristoyloxy-2-pyrrolidinyl, 1-methyl-4-palmitoyloxy-2-pyrrolidinyl, 1-methyl-4-stearoyloxy-2-pyrrolidinyl, 2-piperidyl, 3-piperidyl, 4-piperidyl, 1-methyl-2-piperidyl, 1-methyl-3-piperidyl, 1-methyl-4-piperidyl, 2-morpholinyl or 4-methyl-2-morpholinyl group; and
   A represents an ethylene group,
(16) compounds wherein R¹ represents a hydrogen or fluorine atom,
   R² represents a hydrogen or fluorine atom, R³ represents a methoxy group, R⁴ represents a 2-pyrrolidinyl, 1-methyl-2-pyrrolidinyl, 4-hydroxy-2-pyrrolidinyl, 4-decanoyloxy-2-pyrrolidinyl, 4-lauroyloxy-2-pyrrolidinyl, 1-methyl-4-hydroxy-2-pyrrolidinyl, 1-methyl-4-decanoyloxy-2-pyrrolidinyl or 1-methyl-4-lauroyloxy-2-pyrrolidinyl group; and
   A represents an ethylene group.

Preferable compounds of the formula (I) are exemplified in the following Tables 1 and 2.

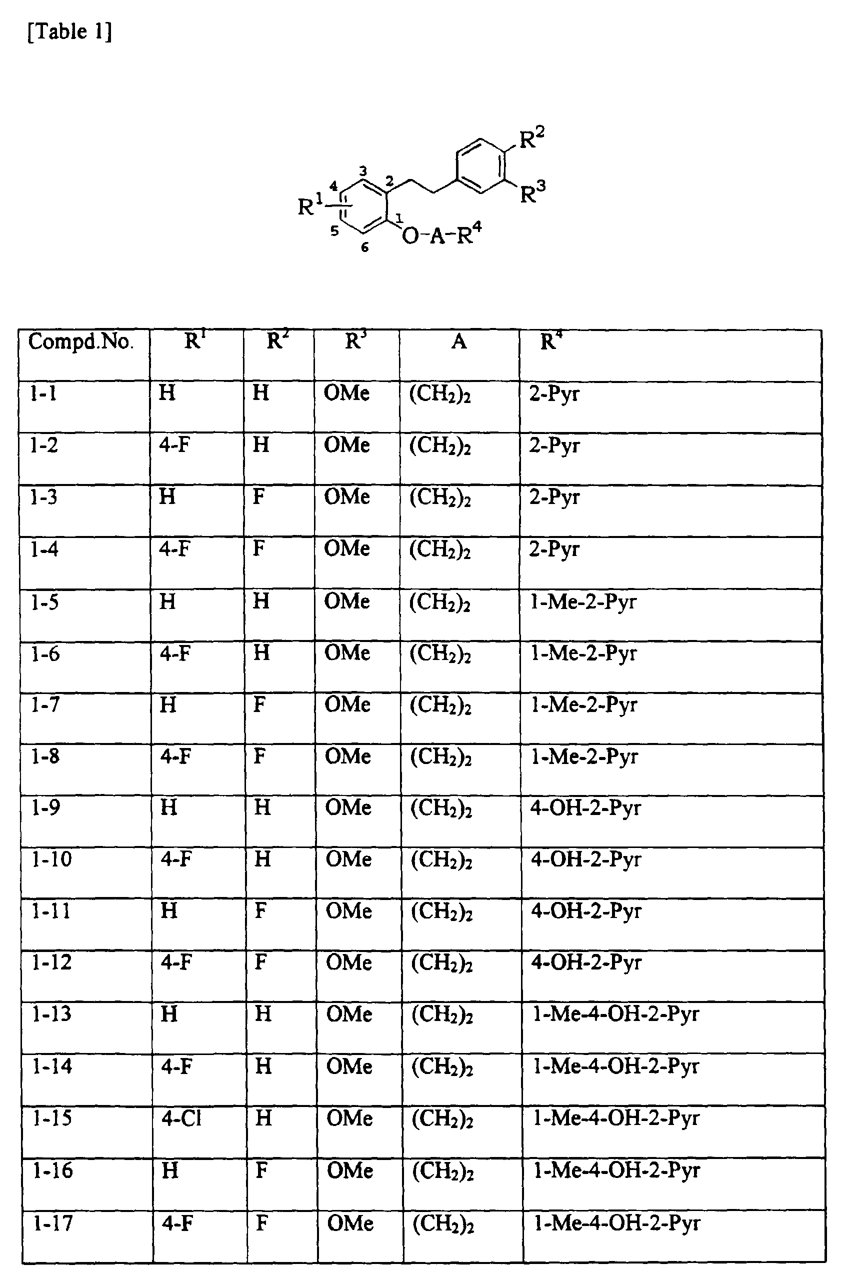

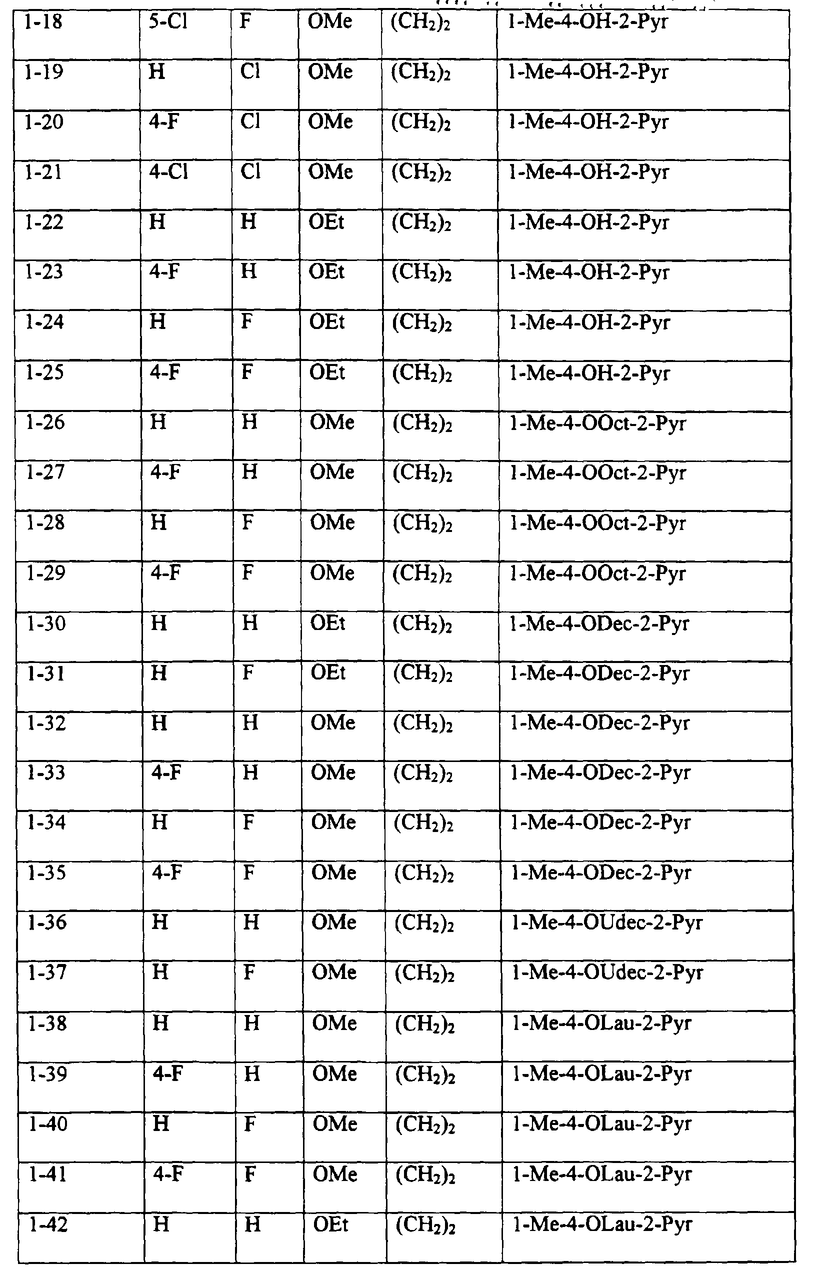

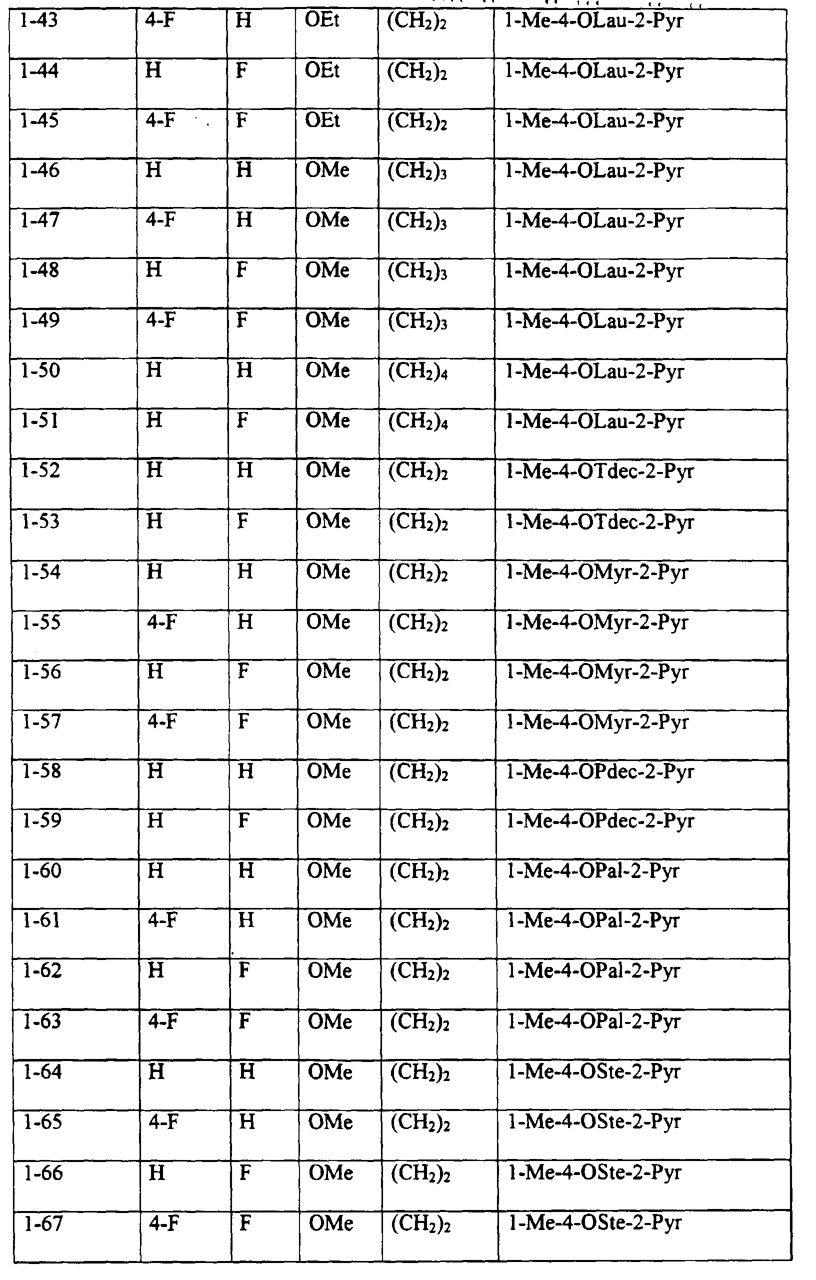

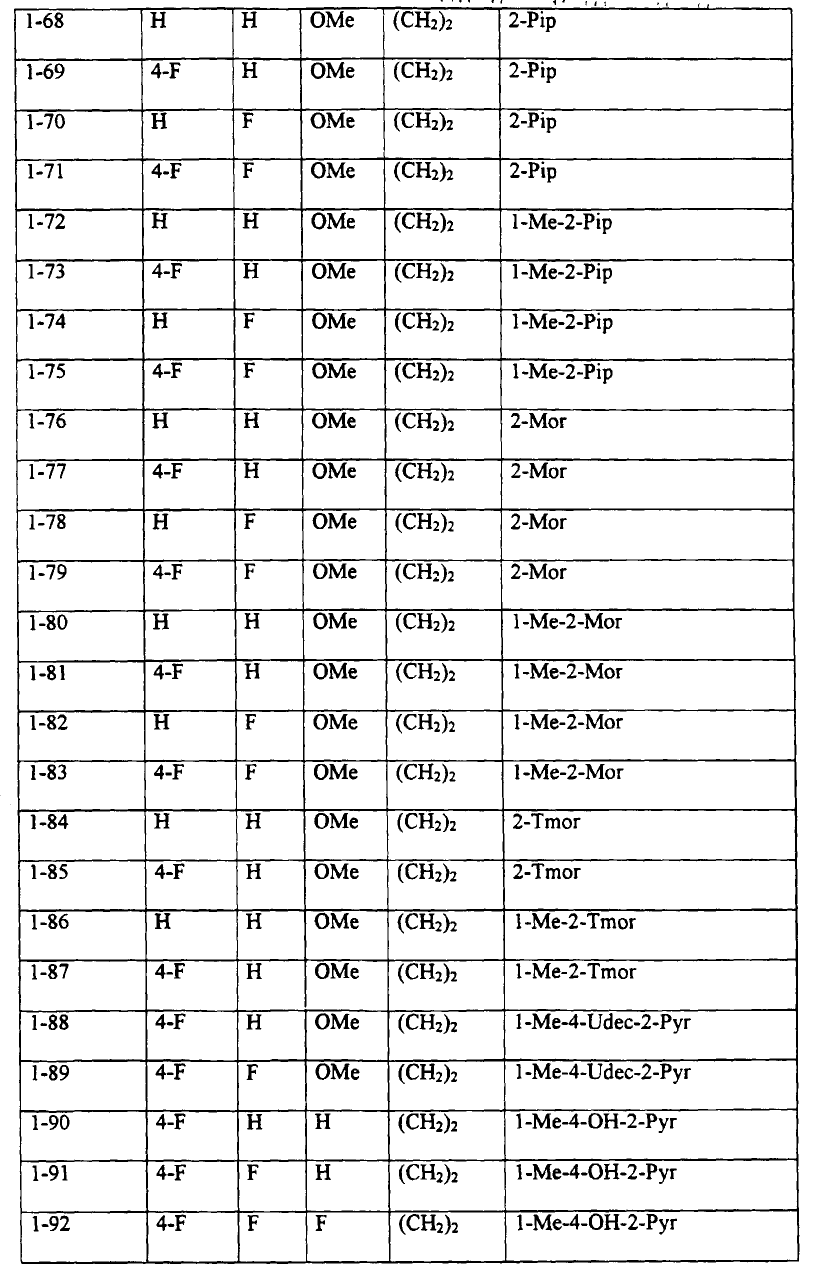

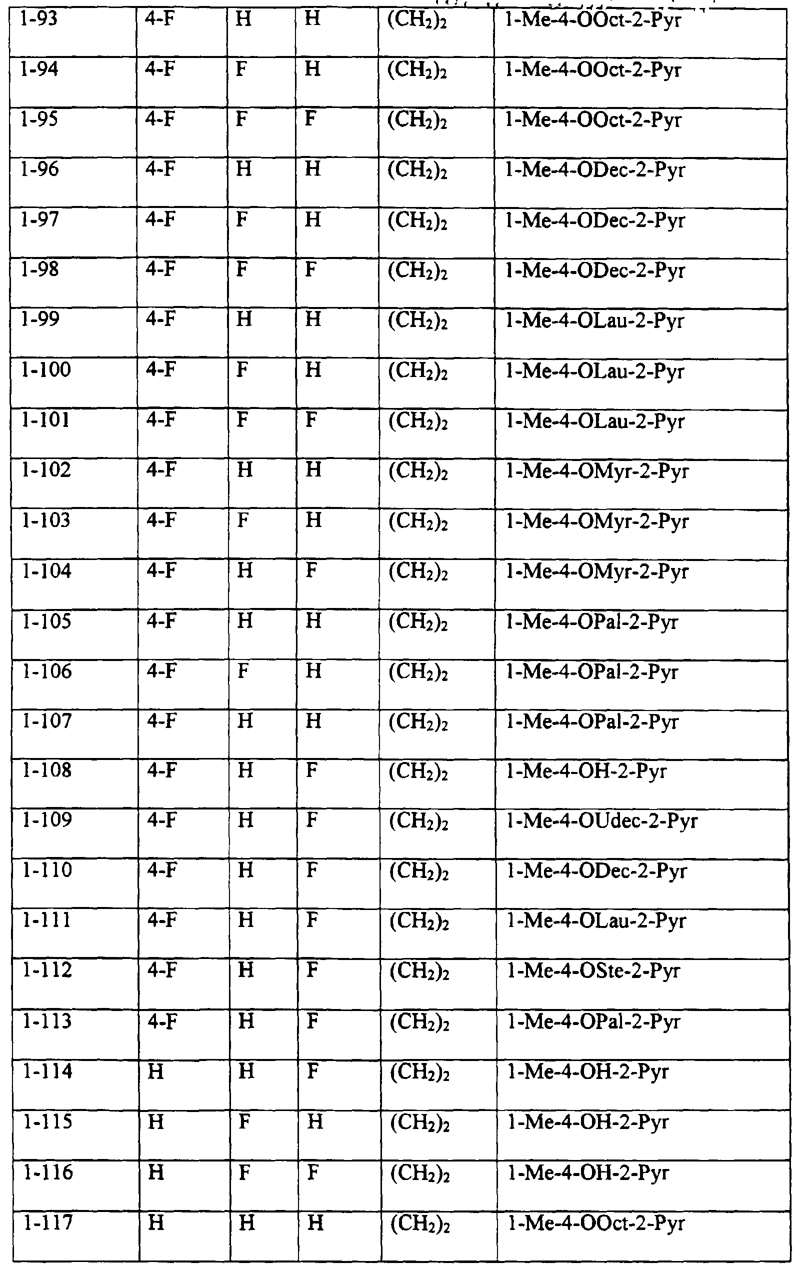

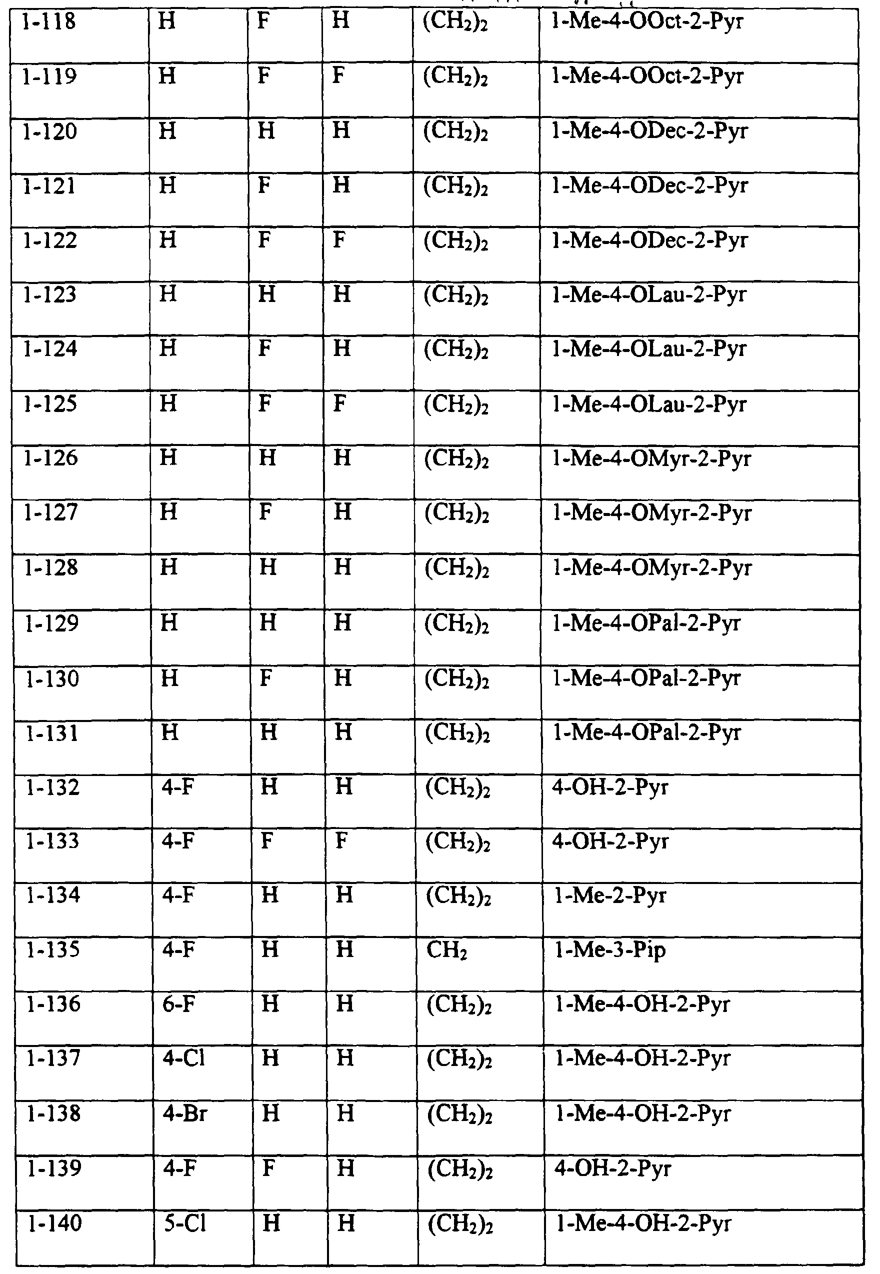

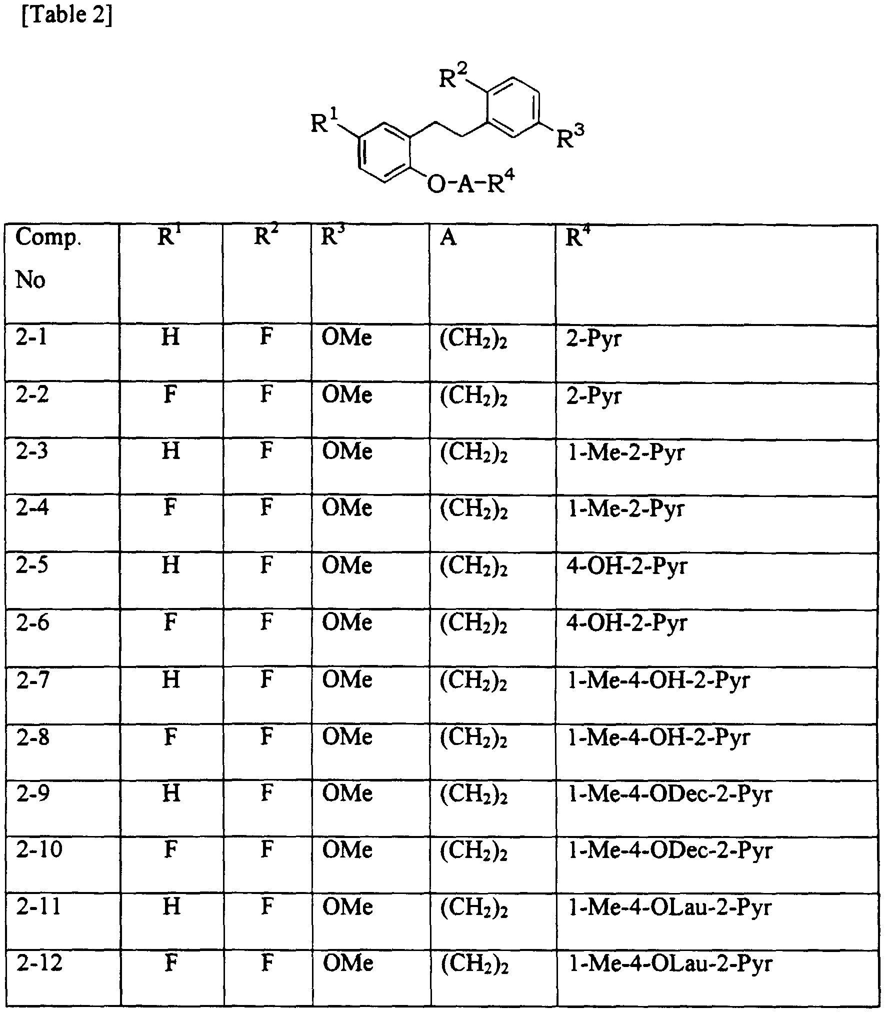

In the above tables, abbreviations mean the following groups, respectively.
Dec: decanoyl group
Et: ethyl group
Lau: lauroyl group
Me: methyl group
Mor: morpholinyl group
Myr: myristoyl group
Oct: octanoyl group
Pal: palmitoyl group.
Pdec: pentadecanoyl group
Pip: piperidyl group
Pyr: pyrrolidinyl group
Ste: stearoyl group
Tdec: tridecanoyl group
Tmor: thiomorpholinyl group
Udec: undecanoyl group

Among the compounds exemplified in the above tables, the following compounds are preferred: Compound Nos. 1-5, 1-6, 1-7, 1-8, 1-13, 1-14, 1-16, 1-17, 1-26, 1-27, 1-28, 1-29, 1-32, 1-33, 1-34, 1-35, 1-36, 1-37, 1-38, 1-39, 1-40, 1-41, 1-60, 1-61, 1-62, 1-63, 1-64, 1-65, 1-66, 1-67, 1-88, 1-89, 1-90, 1-91, 1-92, 1-99, 1-100, 1-101, 1-114, 1-116, 1-132, 1-133, 1-134, 1-135 and 1-139, of which
the compounds of Compound Nos. 1-13, 1-14, 1-16, 1-17, 1-27, 1-28, 1-29, 1-33, 1-34, 1-35, 1-38, 1-39, 1-40, 1-41, 1-60, 1-61, 1-62, 1-63, 1-88, 1-89, 1-90, 1-91, 1-92, 1-100, 1-101, 1-114 and 1-116 are more preferred,
the compounds of Compound Nos. 1-13, 1-14, 1-16, 1-17, 1-33, 1-34, 1-35, 1-38, 1-39, 1-40, 1-41, 1-60, 1-90, 1-91, 1-92, 1-100, 1-101 and 1-114 are still more preferred, and the compounds of:

Compound No. 1-13:
   4-hydroxy-2-[2-[2-[2-(3-methoxyphenyl)ethyl]phenoxy]ethyl]-1-methylpyrrolidine,
Compound No. 1-14:
   2-[2-[4-fluoro-2-[2-(3-methoxyphenyl)ethyl]phenoxy]ethyl]-4-hydroxy-1-methylpyrrolidine,
Compound No. 1-16:
   2-[2-[2-[2-(4-fluoro-3-methoxyphenyl)ethyl]phenoxy]ethyl]-4-hydroxy-1-methylpyrrolidine,
Compound No. 1-17:
   2-[2-[4-fluoro-2-[2-(4-fluoro-3-methoxyphenyl)ethyl]phenoxy]ethyl]-4-hydroxy-1-methylpyrrolidine,
Compound No. 1-33:
   4-decanoyloxy-2-[2-[4-fluoro-2-[2-(3-methoxyphenyl)ethyl]phenoxy]ethyl]-1-methylpyrrolidine,
Compound No. 1-34:
   4-decanoyloxy-2-[2-[2-[2-(4-fluoro-3-methoxyphenyl)ethyl]phenoxy)ethyl]-1-methylpyrrolidine,
Compound No. 1-35:
   4-decanoyloxy-2-[2-[4-fluoro-2-[2-(4-fluoro-3-methoxyphenyl)ethyl]phenoxy]ethyl]-1-methylpyrrolidine,
Compound No. 1-38:
   4-lauroyloxy-2-[2-[2-[2-(3-methoxyphenyl)ethyl]phenoxy]ethyl]-1-methylpyrrolidine
Compound No. 1-39:
   2-[2-[4-fluoro-2-[2-(3-methoxyphenyl)ethyl]phenoxy]ethyl]-4-lauroyloxy-1-methylpyrrolidine,
Compound No. 1-40:
   2-[2-[2-[2-(4-fluoro-3-methoxyphenyl)ethyl]phenoxy)ethyl]-4-lauroyloxy-1-methylpyrrolidine, and
Compound No. 1-41:
   2-[2-[4-fluoro-2-[2-(4-fluoro-3-methoxyphenyl)ethyl]phenoxy]ethyl]-4-lauroyloxy-1-methylpyrrolidine are the most preferred.

The compound of the formula (I) which is an active ingredient of the present invention can be prepared by processes well known in the art (for example, Japanese Patent Application Kokai No. Hei 6-234736, Japanese Patent Application Kokai No. Hei 6-306025 or the like) or can be prepared by the following method.

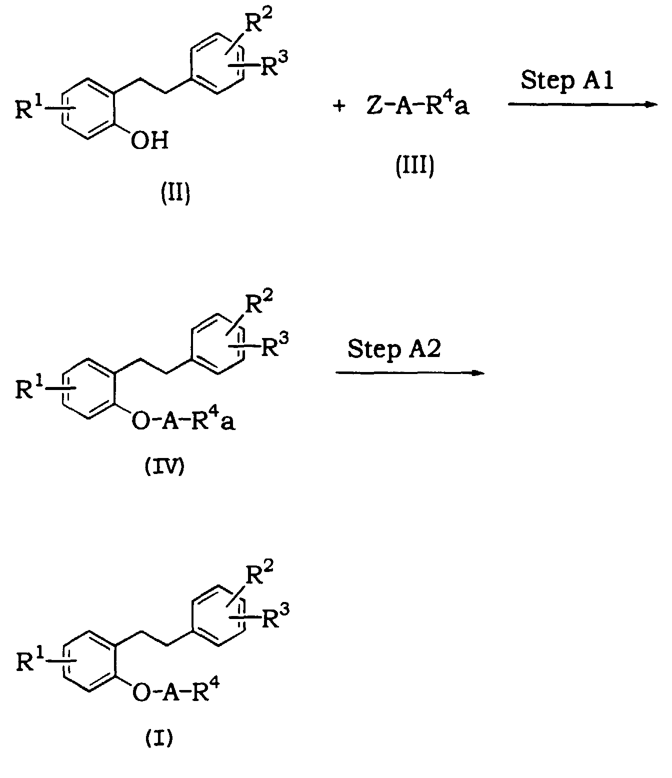

In the above formulae, R¹, R², R³, R⁴ and A are as described above, R⁴a represents a substituted or unsubstituted 5- or 6-membered cyclic amino group which may further contain an oxygen or a sulfur atom (said substituent on a carbon atom is a hydroxy group which may be protected or a C₁-C₂₀ aliphatic acyloxy group which may contain double bonds, and the nitrogen atom of the ring is protected), Z represents a hydroxy group, a halogen atom (preferably, a chlorine, bromine or iodine atom), a C₁-C₆ alkanesulfonyloxy group (examples of the C₁-C₆ alkane moiety of said alkanesulfonyloxy group may be, for example, methane, ethane, propane, butane, pentane or hexane, of which methane or ethane is preferred) or a C₆-C₁₀ arylsulfonyloxy group which may be substituted by C₁-C₆ alkyl, C₁-C₆ alkoxy or halogen.

Examples of the C₁-C₆ alkyl group which is a substituent of the C₆-C₁₀ arylsulfonyloxy group may be, for example, linear or branched C₁-C₆ alkyl groups such as methyl, ethyl, propyl, isopropyl, butyl, isobutyl, s-butyl, t-butyl, pentyl, isopentyl, 2-methylbutyl, neopentyl, 1-ethylpropyl, hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl or 2-ethylbutyl, of which the liner or branched C₁-C₄ alkyl groups are preferred, and a methyl group is the most preferred.

The C₁-C₆ alkoxy group which is a substituent of the C₆-C₁₀arylsulfonyloxy group comprises the above-described "C₁-C₆ alkyl group" bonded to an oxygen atom, and may be, for example, liner or branched C₁-C₆ alkoxy groups such as methoxy, ethoxy, propoxy, isopropoxy, butoxy, isobutoxy, s-butoxy, t-butoxy, pentyloxy, isopentyloxy, 2-methylbutoxy, neopentyloxy, hexyloxy, 4-methylpentyloxy, 3-methylpentyloxy, 2-methylpentyloxy, 3,3-dimethylbutoxy, 2,2-dimethylbutoxy, 1,1-dimethylbutoxy, 1,2-dimethylbutoxy, 1,3-dimethylbutoxy or 2,3-dimethylbutoxy, of which the liner or branched C₁-C₄ alkoxy groups are preferred and a methoxy group is the most preferred.

Examples of the C₆-C₁₀ aryl group of the C₆-C₁₀ arylsulfonyloxy group may be, for example, phenyl or naphthyl groups, of which a phenyl group is preferred.

The protecting group for the hydroxy group of R⁴a may be, for example, cyclic ether groups such as tetrahydofuranyl or tetrahydropyranyl, a methoxymethyl group, a methoxyethoxymethyl group, a C₆-C₁₀ aryl-methyl group, a C₆-C₁₀ aryl-methyloxycarbonyl group, a carbamoyl group, carbamoyl groups substituted by a C₁-C₆ alkyl group such as N-methylcarbamoyl, N-ethylcarbamoyl, N-propylcarbamoyl, N-hexylcarbamoyl, N,N-dimethylcarbamoyl, N,N-diethylcarbamoyl, N,N-diisopropylcarbamoyl, N,N-dibutylcarbamoyl or N-ethyl-N-methylcarbamoyl, or silyl groups having three substituents selected from the group consisting of C₁-C₆ alkyl groups and a phenyl group, such as trimethylsilyl, triisopropylsilyl, triphenylsilyl, dimethylisopropylsilyl, diethylisopropylsilyl, dimethylthexylsilyl, t-butyldimethylsilyl, t-butyldiphenylsilyl or diphenylmethylsilyl, of which a tetrahydropyranyl, methoxymethyl, benzyl, p-methoxybenzyl, p-bromobenzyl, benzyloxycarbonyl, p-methoxybenzyloxycarbonyl, p-bromobenzyloxycarbonyl, N,N-dimethylcarbamoyl or t-butyldimethylsilyl group is preferred.

Examples of the protecting group for the nitrogen atom of the cyclic amino group of R⁴a may be, for example, C₁-C₁₀ alkoxy-carbonyl groups, C₁-C₅ alkanoyl groups, C₆-C₁₀ aryl-methyl groups or C₆-C₁₀ aryl-methyloxycarbonyl groups, of which t-butoxycarbonyl, acetyl, benzyl, p-methoxybenzyl, p-bromobenzyl, benzyloxycarbonyl, p-methoxybenzyloxycarbonyl or p-bromobenzyloxycarbonyl groups are preferred.

Examples of the C₆-C₁₀ aryl moiety of the C₆-C₁₀ aryl-methyl group or C₆-C₁₀ aryl-methyloxycarbonyl group may be, for example, phenyl or naphthyl groups, of which the phenyl group is preferred. The C₆-C₁₀ aryl group may have substituents and examples of the substituent may be, for example, C₁-C₄ alkyl, C₁-C₄ alkoxy or halogen, of which a methyl group, methoxy group, fluorine atom or chlorine atom is preferred.

Examples of the C₁-C₁₀ alkoxy-carbonyl group may be, for example, linear or branched C₁-C₁₀ alkoxy-carbonyl groups such as methoxycarbonyl, ethoxycarbonyl, propoxycarbonyl, isopropoxycarbonyl, butoxycarbonyl, isobutoxycarbonyl, s-butoxycarbonyl, t-butoxycarbonyl, pentyloxycarbonyl, isopentyloxycarbonyl, hexyloxycarbonyl, heptyloxycarbonyl, octyloxycarbonyl, nonyloxycarbonyl or decyloxycarbonyl groups, of which linear or branched C₁-C₈ alkoxy-carbonyl groups are preferred, linear or branched C₁-C₄ alkoxy-carbonyl groups are more preferred and ethoxycarbonyl or t-butoxycarbonyl groups are the most preferred.

Examples of the C₁-C₅ alkanoyl group may be, for example, formyl, acetyl, propionyl, butyryl, isobutyryl, valeryl, isovaleryl or pivaloyl groups, of which an acetyl group is preferred.

The step A1 is a process of preparing the compound of the formula (IV) which is obtained by the reaction of the compound of the formula (II) with the compound of the formula (III).

When Z is a halogen atom, C₁-C₆ alkanesulfonyloxy group or C₆-C₁₀ arylsulfonyloxy group, this reaction is cried out in an inert solvent in the presence of a base. The hydroxyl group contained in the compound (III) is preferred to be protected.

Preferred examples of the base may be, for example, alkali metal carbonates such as sodium carbonate or potassium carbonate, alkali metal bicarbonates such as sodium bicarbonate or potassium bicarbonate, alkali metal fluorides such as sodium fluoride or potassium fluoride, alkali metal hydrides such as sodium hydride, potassium hydride or lithium hydride, alkali metal alkoxides such as sodium methoxide, sodium ethoxide, potassium t-butoxide or lithium methoxide, or organic amines such as pyridine, picoline, triethylamine, N-methylmorpholine or 4-dimethylaminopyridine, of which the alkali metal carbonates, alkali metal fluorides, alkali metal hydrides or alkali metal alkoxides are more preferred.

There is no particular limitation on the nature of the inert solvent to be employed, provided that it has no adverse effect on the reaction, and may be, for example, hydrocarbons such as hexane, benzene or toluene, halogenated hydrocarbons such as methylene chloride, chloroform or 1,2-dichloroethane, ethers such as diethyl ether, tetrahydrofuran or dioxane, ketones such as acetone or methyl ethyl ketone, nitriles such as acetonitrile, amides such as N,N-dimethylacetamide, N,N-dimethylformamide, N-methylpyrrolidone or hexamethylphosphoramide, sulfoxides such as dimethylsulfoxide, or a mixture thereof, of which the ethers, ketones, amides or sulfoxides are preferred.

Although the reaction temperature depends on the nature of compound (II), compound (III), solvent and base, it usually ranges from 0 to 100°C (preferably from 10 to 80°C). The reaction time depends on the reaction temperature and the like, however it ranges from 30 minutes to 48 hours (preferably from 1 to 24 hours).

When Z is a hydroxy group, the reaction is carried out in an inert solvent in the presence of triphenylphosphine and a di-C₁-C₄ alkyl azodicarboxylate such as dimethyl azodicarboxylate or diethyl azodicarboxylate. The hydroxy group contained in compound (III) is preferably protected with a protecting group.

As the inert solvent, those as exemplified above can be employed, of which the aromatic hydrocarbons, halogenated hydrocarbons or ethers are preferred.

Although the reaction temperature depends on the nature of the compound (II), compound (III), solvent and base, it usually ranges from -20 to 100°C (preferably from 10 to 80°C). The reaction time depends on the reaction temperature and the like, however it ranges from 30 minutes to 48 hours (preferably from 1 to 24 hours).

After the completion of the reaction, the target compound (IV) is isolated from the reaction mixture in a usual method. For example, the target compound can be obtained by filtering off insoluble substances from the reaction mixture, if any, as desired, and distilling off the solvent under reduced pressure; or distilling off the solvent under reduced pressure, adding water to the residue, extracting with a water-immiscible organic solvent such as ethyl acetate, drying over anhydrous magnesium sulfate and the like, and then distilling off the organic solvent, if necessary, the product thus obtained can be further purified by a usual method such as recrystallization or column chromatography.

The step A2 is carried out, if necessary and it includes:
reaction (a): deprotection of the protecting group from the protected hydroxy group included in R⁴a,
reaction (b): acylation of the hydroxy group and the like formed by the reaction (a),
reaction (c): deprotection of the protecting group from the protected nitrogen atom included in R⁴a, and
reaction (d): reaction converting the alkoxycarbonyl group included in R⁴a into a methyl group, and alkanoyl group into an alkyl group.

The reaction order of these reactions can be changed, if necessary.

### Reaction (a):

The reaction to remove the protecting group from the protected hydroxy group contained in R⁴a in the reaction (a) depends on the nature of the protecting group. It is carried out according to methods well known in the art.

When the hydroxy group is protected with an arylmethyl or arylmethyloxycarbonyl group, the removal reaction is carried out by reacting a compound (IV) with hydrogen (usually at 1 to 10 atmospheric pressure, preferably 1 to 3 atmospheric pressure) in an inert solvent (preferably, an alcohol such as methanol, ethanol or isopropanol, an ether such as diethyl ether, tetrahydrofuran or dioxane, an aromatic hydrocarbon such as toluene, benzene or xylene, an aliphatic hydrocarbon such as hexane or cyclohexane, an ester such as ethyl acetate or butyl acetate or an aliphatic carboxylic acid such as acetic acid, or a mixture thereof with water) in the presence of a hydrogenation catalyst (preferably, palladium-carbon, Raney nickel, platinum oxide, platinum black, rhodium-aluminum oxide, triphenylphosphine-rhodium chloride or palladium-barium sulfate).

The reaction temperature usually ranges from 0 to 100°C (preferably from 20 to 80°C). The reaction time depends on the reaction temperature and the like, however it usually ranges from 30 minutes to 48 hours (preferably from 1 to 24 hours).

When the hydroxy group is protected with a methoxymethyl, methoxyethoxymethyl or cyclic ether group, the removal reaction is carried out, for example, by reacting a compound (IV) with an acid (for example, an inorganic acid such as hydrochloric acid, nitric acid, hydrochloric acid or sulfuric acid, an organic acid such as acetic acid, trifluoroacetic acid, methanesulfonic acid or p-toluenesulfonic acid, a Lewis acid such as boron trifluoride or a strong-acidic cation exchange resin such as "Dowex 50W" (trade mark), of which the inorganic acid or organic acid is preferred and the hydrochloric acid, sulfuric acid or trifluoroacetic acid is more preferred) in an inert solvent (preferably, a hydrocarbon such as hexane or benzene, a halogenated hydrocarbon such as methylene chloride or chloroform, an ester such as ethyl acetate, a ketone such as acetone or methyl ethyl ketone, an alcohol such as methanol or ethanol, an ether such as diethyl ether, tetrahydrofuran or dioxane, or a mixture thereof with water, of which the ester, ether or halogenated hydrocarbon is preferred).

The reaction temperature usually ranges from -10 to 100°C (preferably from -5 to 50°C). Although the reaction time depends on the reaction temperature and the like, it usually ranges from 5 minutes to 48 hours (preferably 30 minutes to 10 hours).

When a compound (IV) is treated with an acid in the above reaction, it is possible to remove the protecting group and obtain an acid addition salt of the compound (I) at the same time.

After the completion of the reaction, the target compound is isolated from the reaction mixture in a usual method. For example, the target compound can be obtained by filtering off insoluble substances from the reaction mixture, if any, as desired, and distilling off the solvent under reduced pressure; or distilling off the solvent under reduced pressure, adding water to the residue, extracting with a water-immiscible organic solvent such as ethyl acetate, drying over anhydrous magnesium sulfate and the like, and then distilling off the organic solvent, If necessary, the product thus obtained can be further purified by a usual method such as recrystallization or column chromatography.

When the hydroxy group is protected with a substituted or unsubstituted carbamoyl group, the protecting group can be removed by reduction or hydrolysis

In the case of the removal by reduction, the protecting group can be removed, for example, by reacting a compound (IV) with a reducing agent (preferably, a metal hydride reducing agent such as lithium aluminum hydride or lithium borohydride) in an inert solvent (preferably, an ether such as diethyl ether, tetrahydrofuran or dioxane). Although the reaction temperature depends on the solvent and the like, it usually ranges from 0 to 100 °C (preferably from 10 to 80 °C). The reaction time depends on the reaction temperature, however, it usually ranges from 30 minutes to 24 hours (preferably from 1 hour to 16 hours). After the completion of the reaction, the target compound can be obtained from the reaction mixture in a usual method, for example, in a similar manner to that of the step A1.

In the case of the removal by hydrolysis, hydrolysis can be conducted either with an acid or alkali. There is no particular limitation on the nature of the solvent to be employed, provided that it has no adverse effect on the reaction. For example water, an alcohol such as methanol or ethanol or an ether such as dioxane or tetrahydrofuran, or a mixture thereof with water (preferably, water or an alcohol) is usually employed. The acid to be employed is preferably a mineral acid such as hydrochloric acid or sulfuric acid, while the alkali to be employed is preferably a hydroxide of an alkali metal or alkaline earth metal such as sodium hydroxide, potassium hydroxide or barium hydroxide. The reaction temperature depends on the reaction conditions, however, it usually ranges from 0 to 100 °C (preferably 10 to 80 °C). The reaction time depends on the reaction temperature, however, it usually ranges from 30 minutes to 48 hours (preferably 1 to 16 hours). After the completion of the reaction, the target compound can be obtained from the reaction mixture in a usual method, for example, in a similar manner to that of the step A1.

When the hydroxy group is protected with a tri-substituted silyl group, the protecting group can be removed by treating in an inert solvent in the presence of an acid, alkali or fluoride.

In the case of the removal with an acid, examples of the acid to be employed may be, for example, organic acids such as acetic acid, trifluoroacetic acid, citric acid or p-toluenesulfonic acid, inorganic acids such as hydrochloric acid, hydrofluoric acid and sulfuric acid or Lewis acids such as boron trifluoride diethyl etherate (preferably, hydrochloric acid), while examples of the solvent to be employed may be, for example, ethers such as tetrahydrofuran or dioxane, nitriles such as acetonitrile, halogenated hydrocarbons such as methylene chloride or chloroform, alcohols such as methanol or ethanol, hydrocarbons such as hexane or cyclohexane, mixtures thereof, or a mixture of the above-exemplified organic solvent with water (preferably, the ethers and particularly, dioxane). Although the reaction temperature depends on the reaction conditions, it usually ranges from -50 to 100°C (preferably -5 to 80°C). The reaction time depends on the reaction temperature, however, it usually ranges from 10 minutes to 48 hours (preferably, 30 minutes to 16 hours). After the completion of the reaction, the target compound can be obtained from the reaction mixture in a usual method, for example, in a similar manner to that of the step A1.

When the silyl group is removed using an alkali, examples of the alkali to be employed may be, for example, alkali metal hydroxides such as sodium hydroxide or potassium hydroxide or alkali metal carbonates such as potassium carbonate (preferably, the alkali metal carbonates, particularly potassium carbonate). Preferred examples of the inert solvent to be employed may be, for example, alcohols such as methanol or ethanol and water-containing alcohols. Although the reaction temperature depends on the reaction conditions, it usually ranges from 0 to 100°C (preferably 5 to 80°C). The reaction time depends on the reaction temperature, however it usually ranges from 10 minutes to 48 hours (preferably, 30 minutes to 16 hours). After the completion of the reaction, the target compound can be obtained from the reaction mixture in a usual method, for example, in a similar manner to that of the step A1.

When the silyl group is removed in the presence of a fluoride, examples of a reagent for generating fluoride ions may be, for example, tetrabutylammonium fluoride, HF · pyridine complex or potassium fluoride (preferably, tetrabutylammonium fluoride). Examples of the solvent may be, for example, ethers such as tetrahydrofuran or dioxane, nitriles such as acetonitrile, halogenated hydrocarbons such as methylene chloride or chloroform, alcohols such as methanol or ethanol, hydrocarbons such as hexane or cyclohexane, mixtures thereof, or a mixture of the above-exemplified solvent with water (preferably the ethers, particularly tetrahydrofuran). When a water-soluble salt such as potassium fluoride is used in a mixture of water and a water-insoluble solvent, for example, a mixed solvent of methylene chloride and water, the reaction can be accelerated by using a crown ether such as 18-crown-6. Although the reaction temperature depends on the reaction conditions, it usually ranges from -70 to 100°C (preferably -20 to 50°C). The reaction time depends on the reaction temperature, however it usually ranges from 10 minutes to 48 hours (preferably 30 minutes to 16 hours). After the completion of the reaction, the target compound can be obtained from the reaction mixture in a usual method, for example, in a similar manner to that of the step A1.

### Reaction (b):

The acylation of the hydroxy group in the reaction (b) is carried out by a method well known in the art. The acylation is carried out, for example, by an acylating agent in an inert solvent (preferably, an aromatic hydrocarbon such as benzene or toluene, a halogenated hydrocarbon such as methylene chloride or chloroform, an ester such as ethyl acetate, an ether such as tetrahydrofuran or dioxane, a ketone such as methyl ethyl ketone, or an amide such as N,N-dimethylacetamide) in the presence or absence of a base (preferably, an organic tertiary amine such as triethylamine, pyridine, diethylisopropylamine or 4-dimethylaminopyridine).

Examples of the acylating agent to be used may be, for example, C₂-C₂₀ aliphatic acyl halides which may have double bonds, such as acetyl chloride, propionyl chloride, butyryl chloride, butyryl bromide, isobutyryl chloride, valeryl chloride, pivaloyl chloride, hexanoyl chloride, 3,3-dimethylbutyryl chloride, heptanoyl chloride, octanoyl chloride, nonanoyl chloride, decanoyl chloride, lauroyl chloride, myristoyl chloride, palmitoyl chloride, stearoyl chloride, icosanoyl chloride, acryloyl chloride, methacryloyl chloride, crotonoyl chloride or linoleoyl chloride or C₂-C₂₀ carboxylic anhydrides such as acetic formic anhydride, acetic anhydride, propionic anhydride, butanoic anhydride, valeric anhydride, pivalic anhydride, hexanoic anhydride, heptanoic anhydride, octanoic anhydride, nonanoic anhydride, decanoic anhydride, lauric anhydride, myristic anhydride, palmitic anhydride, acrylic anhydride, methacrylic anhydride, crotonic anhydride or linoleic anhydride.

The hydroxy group can also be acylated by reacting the corresponding hydroxy compound with a carboxylic acid (for example, a C₂-C₂₀ aliphatic carboxylic acid which may have double bonds, such as acetic acid, propionic acid, butanoic acid, valeric acid, hexanoic acid, 3,3-dimethylbutanoic acid, heptanoic acid, octanoic acid, nonanoic acid, decanoic acid, lauric acid, myristic acid, palmitic acid, stearic acid, icosanoic acid, acrylic acid, methacrylic acid, crotonic acid or linoleic acid) in a similar manner to that of the step A1, where Z is a hydroxy group.

The reaction temperature usually ranges from -10 to 50°C (preferably from 0 to 30°C). Although the reaction time depends on the reaction temperature and the like, it usually ranges from 15 minutes to 20 hours (preferably from 30 minutes to 10 hours).

After the completion of the reaction, the target compound can be isolated from the reaction mixture in a usual method. For example, after filtering off the insoluble substances in the reaction mixture and/or neutralizing the filtrate or the reaction mixture, if necessary, the target compound is obtained in a similar manner to that of the step A1.

### Reaction (c):

Although the reaction of removing the protecting group from the protected nitrogen atom contained in R⁴a in the reaction (c) depends on the nature of the protecting group, it is carried out by a manner well known in the art.

When the nitrogen atom has been protected with an arylmethyl group or arylmethoxycarbonyl group, the removing reaction is carried out by a similar manner to that described in the reaction (a) of the step A2 where the hydroxy group is protected with an arylmethyl group.

When the nitrogen atom has been protected with a t-butoxycarbonyl group, the removing reaction is carried out in a similar manner to that described in the reaction (a) where the hydroxy group is protected with a methoxymethyl group and the like.

When the nitrogen atom has been protected with an alkoxycarbonyl residue, the protecting group can be removed by hydrolysis with a base (preferably an alkali metal hydroxide such as lithium hydroxide, sodium hydroxide or potassium hydroxide, or an alkali metal carbonate such as sodium carbonate or potassium carbonate) in an inert solvent (preferably, an alcohol such as methanol or ethanol, an ether such as tetrahydrofuran or dioxane, or a mixed solvent of water and the above-exemplified organic solvent).

Although the reaction temperature depends on the nature of the solvent and the like, it usually ranges from 0 to 100°C (preferably, from room temperature to 60 °C). The reaction time depends on the reaction temperature and the like, however, it usually ranges from 30 minutes to 24 hours (preferably, from 1 hour to 16 hours).

After the completion of the reaction, the target compound can be obtained from the reaction mixture by a usual method, for example, in a similar manner to that described in Process A.

### Reaction (d);

The reaction (d) to convert the alkoxycarbonyl group contained in R⁴a into a methyl group or the alkanoyl group into an alkyl group is carried out by reacting with a reducing agent (preferably an aluminum hydride of an alkali metal such as lithium aluminum hydride) in an inert solvent (preferably, an ether such as diethyl ether, tetrahydrofuran or dioxane).

Although the reaction temperature depends on the nature of the solvent and the like, it usually ranges from 0 to 100°C (preferably, from room temperature to 80°C). The reaction time depends on the reaction temperature and the like, however, it usually ranges from 30 minutes to 24 hours (preferably, from 1 to 16 hours).

After the completion of the reaction, the target compound can be obtained from the reaction mixture by a usual method, for example, by a similar manner to that described in the step A1.

The compound (I) can be converted into a pharmacologically acceptable salt thereof by a usual method, for example, by reaction of compound (I) with an acid at room temperature for 5 minutes to 1 hour in an inert solvent (preferably, an ether such as diethyl ether, tetrahydrofuran or dioxane, an alcohol such as methanol or ethanol, or a halogenated hydrocarbon such as methylene chloride or chloroform) and then distilling off the solvent under reduced pressure. The hydrochloride of the compound (I) can also be obtained by adsorbing compound (I) or an acid addition salt thereof on an acidic resin column [for example, CM-Sephadex C-25 (made mark)] and eluting with dilute hydrochloric acid.

The starting material (II) is a known compound or prepared by a known method (for example, Japanese Patent Application Kokai No. Sho 55-20740, Japanese Patent Application Kokai No. Hei 2-304022, Japanese Patent Application Kokai No. Hei 6-234736, Japanese Patent Application Kokai No. Hei 6-306025 and the like).

The compound (I) has excellent pancreatitis inhibitory activity and has low toxicity. It is useful as a remedy or preventive for pancreatitis (preferably, remedy for pancreatitis).

### [Best Modes for Carrying out the Invention]

The present invention will hereafter be described in further detail with reference to pharmaceutical activity tests (test examples), examples and pharmaceutical formulation examples (formulation examples). These examples are not to be construed as limiting the scope of the invention in any way.

### [Test Example 1]

### Inhibitory activity against pancreatitis induced by choline-deficient ethionine supplemented diet

Pancreatitis of a mouse was induced by choline-deficient ethionine supplemented diet (CDE diet). A modified method of Lombardi et al [Lombardi et al, Am. J. Pathol., 79, 465-480 (1975)] was carried out. Briefly, the pancreatitis was induced by feeding an ICR female mouse (Charles River Japan, Inc. three-weeks old) choline deficient diet to which 0.5% (w/w) DL-ethionine was added (CDE diet). Sixty four hours after the feeding with the CDE diet, approximately 0.3 ml of the blood was collected from the heart of the mouse under anesthesia with ether. The collected blood was allowed to stand at room temperature for 2 hours to coagulate, followed by centrifugation (at 12,000 rpm for 10 minutes), to obtain serum. The amylase activity in the serum was measured by an amylase activity assay kit (Amylase B-Test Wako, product of Wako Pure Chemical Industries, Ltd.) and was designated as an index of pancreatitis. The test compound suspended in a 0.5% (w/v) tragacanth solution was administered to mice one hour before the feeding of the CDE diet and was administered twice a day, 6 times in total.

The amylase activities (IU/ml) in serum from mice which received the test compounds are shown in Tables 3, 4 and 5. The control group was fed with the CDE diet to induce pancreatitis but the test compounds were not administered to the control group. The normal group was not fed with the CDE diet but was fed with ordinary food.

**[Table 3]**

| Compound | Dose (mg/kg) | Number of mice | Amylase activity (IU/ml) |
|---|---|---|---|
| Compound of Preparation Example 3 | 10 | 15 | 35.0 |
| | 30 | 15 | 32.1 |
| Control group | - | 20 | 71.7 |
| Normal group | - | 20 | 7.4 |

**[Table 4]**

| Compound | Dose (mg/kg) | Number of mice | Amylase activity (IU/ml) |
|---|---|---|---|
| Compound of Preparation Example 4 | 10 | 10 | 48.4 |
| | 30 | 9 | 30.5 |
| Compound of Preparation Example 2 | 10 | 10 | 53.7 |
| | 30 | 10 | 31.2 |
| Control group | - | 20 | 64.7 |
| Normal group | - | 20 | 6.6 |

**[Table 5]**

| Compound | Dose (mg/kg) | Number of mice | Amylase activity (IU/ml) |
|---|---|---|---|
| Compound of Preparation Example 18 | 10 | 10 | 25.7 |
| | 30 | 10 | 21.8 |
| Control group | - | 10 | 48.0 |
| Normal group | - | 10 | 8.7 |

### [Test Example 2]

### Inhibitory activity against cerulein-induced pancreatitis

Induction of experimental acute pancreatitis in rats was carried out by a modified method of Otsuki et al [Otsuki et al, Dig. Dis. Sci., 35, 242-250 (1990)]. Briefly, cerulein (20 µg/kg) was intraperitoneally administered to Wistar rats (Japan SLC, weight: about 200 g) every one hour, four times in total. Three hours after the final administration, the blood was collected from the jugular vein of the rat under anesthesia with ether. The collected blood was allowed to stand at room temperature for 2 hours to coagulate, followed by centrifugation (at 12,000 rpm for 10 minutes), to obtain serum. The amylase activity of the serum was measured using an amylase activity assay kit (Amylase B-Test Wako, product of Wako Pure Chemical Industries, Ltd.) and was designated as an index of pancreatitis. The test compound suspended in a 0.5% (w/v) tragacanth solution was orally administered 30 minutes before the first injection of the cerulein. The control group is defined as a group in which pancreatitis was induced, but to which the test compounds were not administered.

**[Table 6]**

| Compound | Dose (mg/kg) | Number of rats | Amylase activity (IU/ml) |
|---|---|---|---|
| Compound of Preparation Example 3 | 10 | 15 | 43.6 |
| | 30 | 15 | 34.9 |
| | 100 | 15 | 31.7 |
| Control group | - | 15 | 48.7 |

### [Example 1]

### (2R,4R)-2-[2-[4-Fluoro-2-[2-(3-methoxyphenyl)ethyl]phenoxy]ethyl]-4-hydroxy-1-methylpyrrolidine hydrochloride (Exemplified Compound No. 1-14)

### (a) (2R,4R)-1-Ethoxycarbonyl-2-[2-[4-fluoro-2-[2-(3-methoxyphenyl)ethyl]phenoxy]ethyl]-4-hydroxypyrrolidine

To a solution of 4-fluoro-2-[2-(3-methoxypheyl)ethyl]phenol (399 mg) in N,N-dimethylacetamide (8 ml) was added potassium t-butoxide (363 mg) and (2S,4R)-2-(2-chloroethyl)-1-ethoxycarbonyl-4-hydroxypyrrolidine (718 mg) in an ice-bath. The mixture was stirred at 40°C for 5 hours. To the reaction mixture, ethyl acetate (50 ml) was added and the resulting mixture was washed successively with water and brine. The ethyl acetate layer was dried over anhydrous magnesium sulfate. The solvent was evaporated under reduced pressure. The resulting oil was purified by chromatography column on silica gel (eluent: hexane / ethyl acetate = 3/7), to give 535 mg (yield: 76%) of the title compound as a colorless oil.
NMR spectrum (270 MHz, CDCl₃) δ ppm: 1.1-1.35 (3H, m), 1.75-2.3 (3H, m), 2.3-2.6 (1H, m), 2.75-3.0 (4H, m), 3.4-3.8 (1H, m), 3.45 (1H, dd, J=4.3 and 11.9Hz), 3.79 (3H, s), 3.9-4.3 (5H, m), 4.35-4.5 (1H, m), 6.8-6.9 (6H, m), 7.15-7.25 (1H, m).

### (b) (2R,4R)-2-[2-[4-Fluoro-2-[2-(3-methoxyphenyl)ethyl]phenoxy]ethyl]-4-hydroxy-1-methylpyrrolidine

A solution of (2R,4R)-1-ethoxycarbonyl-2-[2-[4-fluoro-2-[2-(3-methoxyphenyl)ethyl]phenoxy]ethyl]-4-hydroxypyrrolidine (201 mg) from step (a) in tetrahydrofuran (4 ml) was added dropwise to a mixture of lithium aluminum hydride (53 mg) and tetrahydrofuran (4 ml) with stirring in an ice-bath, followed by reflux for 30 minutes. To the reaction mixture in an ice-bath was added sodium sulfate decahydrate to decompose the excess hydride. The insoluble substances in the reaction mixture were filtered off and the filtrate was concentrated by evaporation under reduced pressure. The residue was purified by column chromatography on silica gel (eluent: methylene chloride / methanol = 4/1) to give 139 mg (yield: 80%) of the title compound as a colorless oil.
NMR spectrum (270 MHz, CDCl₃) δ ppm: 1.75-2.2 (3H, m), 2.2-2.4 (1H, m), 2.40 (1H, dd, J=4.5 and 10.8Hz), 2.51 (3H, s), 2.75 -3.05 (5H, m), 3.62 (1H, dd, J=6.0 and 10.8Hz), 3.79 (3H, s), 3.9-4.1 (2H, m), 4.4-4.55 (1H, m), 6.7-6.9 (6H, m), 7.15-7.25 (1H, m).

### (c) (2R,4R)-2-[2-[4-Fluoro-2-[2-(3-methoxyphenyl)ethyl]phenoxy]ethyl]-4-hydroxy-1-methylpyrrolidine hydrochloride

To a solution of (2R,4R)-2-[2-[4-fluoro-2-[2-(3-methoxyphenyl)ethyl]phenoxy]ethyl)-4-hydroxy-1-methylpyrrolidine (246 mg) from step (b) in ethyl acetate (5 ml) was added a 4N hydrogen chloride - ethyl acetate solution (0.25 ml) and the resulting mixture was allowed to stand at room temperature. The crystals thus precipitated were collected by filtration and dried in vacuo, to give 210 mg (yield: 78%) of the title compound as colorless crystals.
Melting point: 128 - 129°C
NMR spectrum (270 MHz, CDCl₃) δ ppm: 2.0-2.2 (1H, m), 2.3-2.65 (2H, m), 2.33 (1H, dd, J=5.9 and 13.8Hz), 2.75-3.0 (4H, m), 2.89 (3H, s), 2.99 (1H, d, J=12.3Hz), 3.78 (3H, s), 3.8-4.2 (4H, m), 4.55-4.7 (1H, m), 6.56-6.8 (4H, m), 6.8-6.9 (2H, m), 7.21 (1H, t, J=7.8Hz).

### [Example 2]

### (2R,4R)-2-[2-[4-Fluoro-2-[2-(4-fluoro-3-methoxypheny)ethyl]phenoxy]ethyl]-4-hydroxy-1-methylpyrrolidine hydrochloride (Exemplified Compound No. 1-17)

### (a) (2R,4R)-1-Ethoxycarbonyl-2-[2-[4-fluoro-2-[2-(4-fluoro-3-methoxyphenyl)ethyl]phenoxy]ethyl]-4-hydroxypyrrolidine

To a solution of 4-fluoro-2-[2-(4-fluoro-3-methoxyphenyl)ethyl]phenol (622 mg) in 7 ml of N,N-dimethylacetamide was added potassium t-butoxide (343 mg) and (2S,4R)-2-(2-chloroethyl)-1-ethoxycarbonyl-4-hydroxypyrrolidine (678 mg) in an ice-bath. The resulting solution was treated in a similar manner to that described in the step (a) of Preparation Example 1. The resulting oil was purified by column chromatography on silica gel (eluent: hexane / ethyl acetate = 2/3) to give 552 mg (yield: 52%) of the title compound as a colorless oil.
NMR spectrum (270 MHz, CDCl₃) δ ppm: 1.1-1.35 (3H, m), 1.7-1.95 (1H, m), 1.96 (1H, dd, J=4.9 and 7.2Hz), 2.05-2.25 (1H, m), 2.25-2.65 (1H, m), 2.75-2.95 (4H, m), 3.45 (1H, dd, J=4.3 and 12.0Hz), 3.45-3.8 (1H, m), 3.83 (3H, s), 3.85-4.05 (1H, m), 4.05-4.3 (3H, m), 4.35-4.5 (1H, m), 6.6-6.9 (5H, m), 6.96 (1H, dd, J=8.0 and 11.3Hz).

### (b) (2R,4R)-2-[2-[4-Fluoro-2-[2-(4-fluoro-3-methoxyphenyl)ethyl]phenoxy]ethyl]-4-hydroxy-1-methylpyrrolidine

(2R,4R)-1-Ethoxycarbonyl-2-[2-[4-fluoro-2-[2-(4-fluoro-3-methoxyphenyl)ethyl]phenoxy]ethyl]-4-hydroxypyrrolidine (551 mg) from step (a) was reacted with lithium aluminum hydride (140 mg) in tetrahydrofuran (20 ml) and treated in a similar manner to that described in the step (b) of Preparation Example 1. The resulting residue was purified by column chromatography on silica gel (eluent: methylene chloride / methanol = 3/2) to give 405 mg (yield: 84%) of the title compound as a colorless oil.
NMR spectrum (270 MHz, CDCl₃) δ ppm: 1.65-2.1 (3H, m), 2.1-2.3 (1H, m), 2.25 (1H, dd, J=5.2 and 10.3Hz), 2.9 (3H, s), 2.6-2.8 (1H, m), 2.8-3.0 (4H, m), 3.50 (1H, dd, J=6.2 and 10.3Hz), 3.84 (3H, s), 3.85-4.05 (2H, m), 4.35-4.5 (1H, m), 6.65-7.05 (6H, m).

### (c) (2R,4R)-2-[2-[4-Fluoro-2-[2-(4-fluoro-3-methoxyphenyl]ethyl]phenoxyl]ethyl]-4-hydroxy-1-methylpyrrolidine hydrochloride

To a solution of (2R,4R)-2-[2-[4-fluoro-2-[2-(4-fluoro-3-methoxyphenyl)ethyl]phenoxy]ethyl]-4-hydroxy-1-methylpyrrolidine (399 mg) from step (b) in ethyl acetate (5 ml) was added a 4N hydrogen chloride - ethyl acetate solution (0.38 ml) to precipitate crystals. The solvent of the mixture was distilled off under reduced pressure. After the solid thus obtained was dissolved in a small amount (about 0.5 ml) of methylene chloride, to the resulting solution was added ethyl acetate (5 ml). The solution was allowed to stand at room temperature. The crystals thus precipitated were collected by filtration and then dried in vacuo, to give 359 mg (yield: 82%) of the title compound as colorless crystals.
Melting point: 128 - 130°C
NMR spectrum (400 MHz, CD₃SOCD₃ + D₂O) δ ppm: 1.8-2.0 (1H, m), 2.0-2.2 (1H, m), 2.20 (1H, dd, J=6.0 and 13.7Hz), 2.4-2.55 (1H, m), 2.7-3.0 (4H, m), 2.89 (3H, s), 2.97 (1H, d, J=12.5Hz), 3.6-3.9 (2H, m), 3.80 (3H, s), 3.95-4.15 (2H, m), 4.3-4.45 (1H, m), 6.7-6.8 (1H, m), 6.9-7.15 (5H, m).

### [Example 3]

### (2R,4R)-4-Lauroyloxy-2-[2-[2-[2-(3-methoxyphenyl)ethyl]phenoxy]ethyl]-1-methylpyrrolidine hydrochloride

### (a) (2R,4R)-4-Lauroyloxy-2-[2-[2-[2-(3-methoxyphenyl)ethyl]phenoxyl]ethyl]-1-methylpyrrolidine (Exemplified Compound No. 1-38)

To a solution of (2R,4R)-4-hydroxy-2-[2-[2-[2-(3-methoxyphenyl)ethyl]phenoxy]ethyl]-1-methylpyrrolidine (388 mg) prepared according to Example 68 of Japanese Patent Application Kokai No. Hei 6-234736 in pyridine (5 ml) were added lauric anhydride (543 mg) and 4-dimethylaminopyridine (40 mg). The mixture was stirred at room temperature for 4 hours. The reaction mixture was concentrated by evaporation under reduced pressure and the residue was extracted with ethyl acetate. The ethyl acetate layer was dried over magnesium sulfate. The solvent was evaporated under reduced pressure to give an oil. The resulting oil was purified by column chromatography on silica gel (eluent: ethyl acetate), to give 549 mg (yield: 94%) of the title compound as a colorless oil.
NMR spectrum (270 MHz, CDCl₃) δ ppm: 0.88 (3H, t, J=6.7Hz), 1.15-1.4 (16H, m), 1.55-1.8 (3H, m), 1.85-2.15 (2H, m), 2.2-2.4 (2H, m), 2.21 (2H, t, J=7.6Hz), 2.37 (3H, s), 2.55-2.7 (1H, m), 2.8-3.0 (4H, m), 3.58 (1H, dd, J=6.6 and 10.6Hz), 3.78 (3H, s), 3.9-4.15 (2H, m), 5.05-5.2 (1H, m), 6.7-6.95 (5H, m), 7.1-7.25 (3H, m).

### (b) (2R,4R)-4-Lauroyloxy-2-[2-[2-[2-(3-methoxypheny)lethyl]phenoxy]ethyl]-1-methylpyrrolidine hydrochloride

To a solution of (2R,4R)-4-lauroyloxy-2-[2-[2-[2-(3-methoxyphenyl)ethyl]phenoxy]ethyl]-1-methylpyrrolidine (535 mg) from step (a) in dioxane (6 ml) was added a 4N hydrogen chloride - dioxane solution (0.75 ml), followed by concentration by evaporation under reduced pressure. To a solution of the concentrate in ethyl acetate (1 ml) was added diethyl ether (10 ml). The mixture was stirred. The crystals thus precipitated were collected by filtration and dried in vacuum, to give 446 mg (yield: 78%) of the title compound as colorless crystals.
Optical rotation: [α]_{D} -2.1° (c=1.19, methanol)
Melting point: 73 - 74°C
NMR spectrum (270 MHz, CDCl₃) δ ppm: 0.88 (3H, t, J=6.6Hz), 1.1-1.35 (16H, m), 1.4-1.6 (2H, m), 2.13 (2H, t, J=7.6Hz), 2.25-2.5 (2H, m), 2.5-2.7 (2H, m), 2.75-2.95 (5H, m), 2.85 (3H, s), 3.65-3.8 (1H, m), 3.77 (3H, s), 3.9-4.05 (1H, m), 4.2-4.35 (1H, m), 4.34 (1H, dd, J=5.6 and 13.5Hz), 5.3-5.4 (1H, m), 6.7-6.8 (3H, m), 6.85 (1H, d, J=8.0Hz), 6.94 (1H, t, J=7.3Hz), 7.15-7.3 (3H, m).

### [Example 4]

### (2R,4R)-2-[2-[2-[2-(4-Fluoro-3-methoxyphenyl)ethyl]phenoxy]ethyl]-4-hydroxy-1-methylpyrrolidine hydrochloride (Exemplified Compound No. 1-16)

### (a) (2R,4R)-1-Ethoxycarbonyl-2-[2-[2-[2-(4-fluoro-3-methoxyphenyl)ethyl]phenoxy]ethyl]-4-hydroxypyrrolidine

To a solution of 2-[2-(4-fluoro-3-methoxyphenyl)ethyl]phenol (170 mg) in N,N-dimethylacetamide (4 ml) was successively added potassium t-butoxide (101 mg) and a solution of (2S,4R)-2-(2-chloroethyl)-1-ethoxycarbonyl-4-hydroxypyrrolidine (199 mg) in N,N-dimethylacetamide (2 ml) in an ice-bath. The mixture was stirred at room temperature for 3 hours and then at 40°C for 12 hours. To the reaction mixture, ethyl acetate (150 ml) and 1N hydrochloric acid (60 ml) were added. The ethyl acetate layer was washed successively with water and brine, dried over anhydrous magnesium sulfate and concentrated by evaporation under reduced pressure, to give an oil. The oil was purified by column chromatography on silica gel (eluent: hexane / ethyl acetate = 7/3), to give 184 mg (yield: 62%) of the title compound as a colorless oil.
NMR spectrum (270 MHz, CDCl₃) δ ppm: 1.1-1.4 (3H, m), 1.75-2.1 (2H, m), 2.1-2.3 (1H, m), 2.3-2.7 (1H, m), 2.75-3.0 (4H, m), 3.4-3.9 (1H, m), 3.46 (1H, dd, J=4.2 and 11.9Hz), 3.83 (3H, s), 3.9-4.3 (5H, m), 4.35-4.5 (1H, m), 6.65-7.25 (7H, m).

### (b) (2R,4R)-2-[2-[2-[2-(4-Fluoro-3-methoxyphenyl)ethyl]phenoxy]ethyl]-4-hydroxy-1-methylpyrrolidine

(2R,4R)-1-Ethoxycarbonyl-2-[2-[2-[2-(4-fluoro-3-methoxyphenyl)ethyl]phenoxy]ethyl]-4-hydroxypyrrolidine (180 mg) from step (a) was reacted with lithium aluminum hydride (47 mg) in tetrahydrofuran (10 ml) and treated in a similar manner to that described in the step (b) of Preparation Example 1. The residue thus obtained was purified by column chromatography on silica gel (eluent: methylene chloride / methanol = 4/1), to give 101 mg (yield: 65%) of the title compound as a colorless oil.
NMR spectrum (270 MHz, CDCl₃) δ ppm: 1.6-1.8 (1H, m), 1.8-2.0 (2H, m), 2.15-2.3 (1H, m), 2.20 (1H, dd, J=10.1 and 6.4Hz), 2.37 (3H, s), 2.55-2.75 (1H, m), 2.75-3.0 (4H, m), 3.46 (1H, dd, J=6.3 and 10.1Hz), 3.82 (3H, s), 3.9-4.1 (2H, m), 4.3-4.5 (1H, m), 6.65-6.8 (2H, m), 6.8-7.25 (5H, m).

### (c) (2R,4R)-2-[2-[2-[2-(4-Fluoro-3-methoxyphenyl)ethyl]phenoxy]ethyl]-4-hydroxy-1-methylpyrrolidine hydrochloride

To a solution of (2R,4R)-2-[2-[2-[2-(4-fluoro-3-methoxyphenyl)ethyl]phenoxy]ethyl]-4-hydroxy-1-methylpyrrolidine (101 mg) from step (b) in ethyl acetate (3 ml) was added a 4N hydrogen chloride - ethyl acetate solution (0.10 ml). The mixture was concentrated by evaporation under reduced pressure. The resulting oil was dissolved in a small amount of ethyl acetate and allowed to stand at room temperature, to give crystals. The resulting crystals were collected by filtration and dried, to afford 86 mg (yield: 78%) of the title compound as colorless crystals.
Melting point: 98 - 100°C
NMR spectrum (270 MHz, CDCl₃) δ ppm: 2.0-2.2 (1H, m), 2.3-2.65 (2H, m), 2.33 (1H, dd, J=5.8 and 14.0Hz), 2.75-3.0 (4H, m), 2.87 (3H, s), 2.99 (1H, d, J=12.2Hz), 3.7-3.9 (1H, m), 3.81 (3H, s), 3.95-4.25 (3H, m), 4.55-4.7 (1H, m), 6.6-6.75 (2H, m), 6.8-7.25 (5H, m).

### [Example 5]

### (2R,4R)-2-[2-[4-Fluoro-2-[2-(4-fluorophenyl)ethyl]phenoxyl]ethyl]-4-hydroxy-1-methyloyrrolidine hydrochloride (Exemplified Compound No. 1-91)

### (a) (2R,4R)-1-t-Butoxycarbonyl-4-t-butyldimethylsilyloxy-2-[2-4-fluoro-2-[2-(4-fluorophenyl)ethyl]phenoxy]ethyl]pyrrolidine

To a solution of 4-fluoro-2-[2-(4-fluorophenyl)ethyl]phenol (248 mg) in N,N-dimethylacetamide (10 ml) were added potassium t-butoxide (125 mg) and (2S,4R)-1-t-butoxycarbonyl-4-t-butyldimethylsilyloxy-2-(2-chloroethyl)pyrrolidine (405 mg) in an ice-bath. The mixture was stirred at room temperature for 3 hours. Ethyl acetate (150 ml) was added to the reaction mixture. The resulting mixture was washed successively with water and brine. The ethyl acetate layer was dried over anhydrous magnesium sulfate and concentrated by evaporation under reduced pressure, to afford oil. The oil was purified by column chromatography on silica gel (eluent: hexane / ethyl acetate = 4/1) to give 433 mg (yield: 73%) of the title compound as a colorless oil.
NMR spectrum (270 MHz, CDCl₃) δ ppm: 0.02 (3H, s), 0.03 (3H, s), 0.84 (9H, s), 1.46 (9H, s), 1.7-1.9 (2H, m), 2.0-2.15 (1H, m), 2.25-2.5 (1H, m), 2.75-2.95 (4H, m), 3.3-3.7 (2H, m), 3.9-4.2 (3H, m), 4.25-4.4 (1H, m), 6.7-7.0 (5H, m), 7.05-7.2 (2H, m).

### (b) (2R,4R)-2-[2-[4-Fluoro-2-[2-(4-fluorophenyl)ethyl]phenoxy]ethyl]-4-hydroxy-1-methylpyrrolidine

To a solution of (2R,4R)-1-t-butoxycarbonyl-4-t-butyldimethylsilyloxy-2-[2-[4-fluoro-2-[2-(4-fluorophenyl)ethyl]phenoxy]ethyl]pyrrolidine (398 mg) from step (a) in tetrahydrofuran (10 ml) was added dropwise a mixture of lithium aluminum hydride (81 mg) and tetrahydrofuran (10 ml) with stirring in an ice-bath, followed by reflux for one hour. To the reaction mixture in an ice-bath was added sodium sulfate decahydrate to decompose the excess hydride. The insoluble substances were filtered off and the filtrate was concentrated by evaporation under reduced pressure. The residue was purified by column chromatography on silica gel (eluent: methylene chloride / methanol = 7/3), to give 151 mg (yield: 59%) of the title compound as a colorless oil.
NMR spectrum (270 MHz, CDCl₃) δ ppm: 1.6-1.8 (1H, m), 1.8-2.0 (2H, m), 2.1-2.3 (2H, m), 2.34 (3H, s), 2.6-2.75 (1H, m), 2.8-2.95 (4H, m), 3.49 (1H, dd, J=6.3 and 10.2Hz), 3.85-4.05 (2H, m), 4.35-4.5 (1H, m), 6.7-6.9 (3H, m), 6.9-7.0 (2H, m), 7.05-7.2 (2H, m).

### (c) (2R,4R)-2-[2-[4-Fluoro-2-[2-(4-fluorophenyl)ethyl]phenoxy]ethyl]-4-hydroxy-1-methylpyrrolidine hydrochloride

To a solution of (2R,4R)-2-[2-[4-fluoro-2-[2-(4-fluorophenyl)ethyl]phenoxy]ethyl]-4-hydroxy-1-methylpyrrolidine (138 mg) from step (b) in ethyl acetate (4 ml) was added a 4N hydrogen chloride - ethyl acetate solution (0.15 ml), followed by concentration. The resulting oil was dissolved in ethyl acetate (5 ml). The resulting solution was allowed to stand at room temperature, to afford crystals. The crystals were collected by filtration and dried in vacuo, to give 66 mg (yield: 43%) of the title compound as colorless crystals.
Melting point: 70 - 73°C
NMR spectrum (270 MHz, CDCl₃) δ ppm: 2.0-2.2 (1H, m), 2.25-2.65 (3H, m), 2.78 (4H, s), 2.84 (3H, s), 2.99 (1H, d, J=12.4Hz), 3.7-3.9 (1H, m), 3.9-4.2 (3H, m), 4.55-4.7 (1H, m), 6.7-7.05 (5H, m), 7.05-7.2 (2H, m).

### [Example 6]

### (2R,4R)-2-[2-[4-Fluoro-2-[2-(4-fluorophenyl)ethyl]phenoxy]ethyl]-4-hvdroxypyrrolidine hydrochloride (Exemplified Compound No. 1-139)

### (a) (2R,4R)-1-t-Butoxycarbonyl-2-[2-[4-fluoro-2-[2-(4-fluorophenyl)ethyl]phenoxy]ethyl]-4-hydroxypyrrolidine

To a solution of 4-fluoro-2-[2-(4-fluorophenyl)ethyl]phenol (687 mg) in N,N-dimethylacetamide (12 ml) was added potassium t-butoxide (212 mg) in an ice-bath, followed by stirring for 10 minutes. (2S,4R)-2-(2-Chloroethyl)-1-t-butoxycarbonyl-4-t-butyldimethylsilyloxypyrrolidine (687 mg) was added to the resulting solution. The mixture was stirred at room temperature for 14 hours. After potassium t-butoxide (135 mg) was added to the reaction mixture, the resulting mixture was stirred at 40°C for 4 hours, and ethyl acetate (300 ml) was added to the reaction mixture, followed by washing successively with water and brine. The ethyl acetate layer was dried over anhydrous magnesium sulfate and concentrated by evaporation under reduced pressure, to afford oil. The oil was purified by column chromatography on silica gel (eluent: hexane / ethyl acetate = 2/3), to give 571 mg (yield: 74%) of the title compound as a colorless oil.
NMR spectrum (270 MHz, CDCl₃) δ ppm: 1.45 (9H, s), 1.7-2.05 (2H, m), 2.1-2.25 (1H, m), 2.3-2.55 (1H, m), 2.85 (4H, s), 3.4-3.75 (1H, m), 3.42 (1H, dd, J=4.4 and 11.9Hz), 3.9-4.05 (2H, m), 4.1-4.25 (1H, m), 4.35-4.5 (1H, m), 6.7-6.9 (3H, m), 6.9-7.0 (2H, m), 7.05-7.2 (2H, m).

### (b) (2R,4R)-2-[2-[4-Fluoro-2-[2-(4-fluorophenyl)ethyl]phenoxy]ethyl]-4-hydroxypyrrolidine hydrochloride

To a solution of (2R,4R)-1-t-butoxycarbonyl-2-[2-[4-fluoro-2-[2-(4-fluorophenyl)ethyl]phenoxy]ethyl]-4-hydroxypyrrolidine (570 mg) from step (a) in ethyl acetate (5 ml) was added a 4N hydrogen chloride - ethyl acetate solution (5 ml), followed by stirring at room temperature for 30 minutes, to afford crystals. The crystals were collected by filtration, washed with ethyl acetate and then dried in vacuo, to give 381 mg (yield: 78%) of the title compound as colorless crystals.
Melting point: 186 - 187°C
NMR spectrum (270 MHz, DMSO) δ ppm: 1.65-1.85 (1H, m), 2.0-2.4 (3H, m), 2.82 (4H, s), 3.01 (1H, d, J=12.2Hz), 3.3-3.45 (1H, m), 3.8-4.0 (1H, m), 4.06 (1H, t, J=6.1Hz), 4.35-4.45 (1H, m), 5.41 (1H, d, J=3.0Hz), 6.9-7.15 (5H, m), 7.2-7.3 (2H, m).

### [Example 7]

### (2R,4R)-2-[2-[4-Fluoro-2-(2-phenylethyl)phenoxy]ethyl]-4-hydroxypyrrolidine hydrochloride (Exemplified Compound No. 1-132)

### (a) (2R,4R)-1-t-butoxycarbonyl-4-t-butyldimethylsilyloxy-2-[2-[4-fluoro-2-(2-phenylethyl)phenoxylethyl]pyrrolidine

4-Fluoro-2-(2-phenylethyl)phenol (1090 mg) was reacted with potassium t-butoxide (566 mg) and (2S,4R)-2-(2-bromoethyl)-1-t-butoxycarbonyl-4-t-butyldimethylsilyloxypyrrolidine (1870 mg) in N,N-dimethylacetamide (10 ml) and treated in a similar manner to that described in the step (a) of Preparation Example 6, to afford oil. The oil was purified by column chromatography on silica gel (eluent: hexane / ethyl acetate = 5/1), to give 2090 mg (yield: 84%) of the title compound as a colorless oil.
NMR spectrum (270 MHz, CDCl₃) δ ppm: 0.02 (3H, s), 0.03 (3H, s), 0.84 (9H, s), 1.45 (9H, s), 1.7-1.95 (2H, m), 2.0-2.15 (1H, m), 2.25-2.5 (1H, m), 2.8-2.95 (4H, m), 3.3-3.65 (1H, m), 3.35 (1H, dd, J=4.5 and 11.0Hz), 3.85-4.2 (3H, m), 4.25-4.4 (1H, m), 6.7-6.9 (3H, m), 7.15-7.35 (5H, m).

### (b) (2R,4R)-2-[2-[4-Fluoro-2-(2-phenylethyl)phenoxy]ethyl]-4-hydroxypyrrolidine hydrochloride

To a solution of (2R,4R)-1-t-butoxycarbonyl-4-t-butyldimethylsilyloxy-2-[2-[4-fluoro-2-(2-phenylethyl)phenoxy]ethyl]pyrrolidine (600 mg) from step (a) in 5 ml of dioxane was added a 4N hydrogen chloride - dioxane solution (5 ml). The mixture was allowed to stand at room temperature for one hour. The solvent was distilled off under reduced pressure to give a solid. The solid was dissolved in a small amount of methylene chloride and methanol, followed by the addition of ethyl acetate (10 ml) to afford crystals. The crystals were collected by filtration and dried in vacuo, to give 270 mg (yield: 67%) of the title compound as colorless crystals.
Melting point: 151 - 152°C
NMR spectrum (270 MHz, CD₃OD) δ ppm: 1.8-2.0 (1H, m), 2.1-2.4 (3H, m), 2.8-3.0 (4H, m), 3.22 (1H, d, J=12.4Hz), 3.46 (1H, dd, J=4.1 and 12.4Hz), 4.0-4.2 (3H, m), 4.5-4.6 (1H, m), 6.8-7.0 (3H, m), 7.1-7.3 (5H, m).

### [Example 8]

### (2R,4R)-2-[2-[4-Fluoro-2-(2-phenylethyl)phenoxy]ethyl]-4-hydroxy-1-methylpyrrolidine hydrochloride (Exemplified Compound No. 1-90)

### (a) (2R,4R)-1-t-butoxycarbonyl-2-[2-[4-fluoro-2-(2-phenylethyl)phenoxy]ethyl]-4-hydroxypyrrolidine

To a solution of (2R,4R)-1-t-butoxycarbonyl-4-t-butyldiinethylsilyloxy-2-[2-[4-fluoro-2-(2-phenylethyl)phenoxy]ethyl]pyrrolidine (1490 mg) from step (a) of Preparation Example 7 in tetrahydrofuran (15 ml) was added tetrabutylammonium fluoride (0.79 ml), followed by stirring at room temperature for 0.5 hours. The reaction mixture was concentrated by evaporation under reduced pressure. The oily residue was purified by column chromatography on silica gel (eluent: hexane / ethyl acetate = 1/1), to give 1115 mg (yield: 95%) of the title compound as a colorless solid.
NMR spectrum (270 MHz, CDCl₃) δ ppm: 1.45 (9H, s), 1.7-2.05 (2H, m), 2.05-2.25 (1H, m), 2.3-2.55 (1H, m), 2.88 (4H, s), 3.4-3.75 (1H, m), 3.42 (1H, dd, J=4.4 and 11.9Hz), 3.9-4.05 (2H, m), 4.05-4.25 (1H, m), 4.3-4.45 (1H, m), 6.7-6.9 (3H, m), 7.1-7.35 (5H, m).

### (b) (2R,4R)-2-[2-[4-Fluoro-2-(2-phenylethyl)phenoxy]ethyl]-4-hydroxy-1-methylpyrrolidine

(2R,4R)-1-t-Butoxycarbonyl-2-[2-[4-fluoro-2-(2-phenylethyl)phenoxy]ethyl]-4-hydroxypyrrolidine (1115 mg) from step (a) was reacted with lithium aluminum hydride (200 mg) in tetrahydrofuran (20 ml) and treated in a similar manner to that described in the step (b) of Preparation Example 1. The residue thus obtained was purified by column chromatography on silica gel (eluent: methylene chloride / methanol = 5/1), to give 540 mg (yield: 61%) of the title compound as a colorless solid.
NMR spectrum (270 MHz, CDCl₃) δ ppm: 1.65-2.3 (4H, m), 2.30 (1H, dd, J=4.8 and 10.5Hz), 2.44 (3H, s), 2.7-2.95 (1H, m), 2.88 (4H, s), 3.55 (1H, dd, J=6.1 and 10.5Hz), 3.85-4.1 (2H, m), 4.35-4.5 (1H, m), 6.7-6.9 (3H, m), 7.1-7.25 (5H, m).

### (c) (2R,4R)-2-[2-[4-Fluoro-2-(2-phenylethyl)phenoxy]ethyl]-4-hydroxy-1-methylpyrrolidine hydrochloride

To a solution of (2R,4R)-2-[2-[4-fluoro-2-(2-phenylethyl)phenoxy]ethyl]-4-hydroxy-1-methylpyrrolidine (540 mg) from step (b) in ethyl acetate (5 ml) was added a 4N hydrogen chloride - ethyl acetate solution (0.60 ml) to afford crystals. The crystals were collected by filtration and dried in vacuo, to give 515 mg (yield: 86%) of the title compound as colorless crystals.
Melting point: 121 - 122°C
NMR spectrum (400 MHz, DMSO + D₂O) δ ppm: 2.0-2.15 (1H, m), 2.25-2.6 (2H, m), 2.33 (1H, dd, J=5.8 and 13.9Hz), 2.85 (4H, s), 2.87 (3H, s), 3.00 (1H, d, J=12.5Hz), 3.7-4.2 (4H, m), 4.5-4.65 (1H, m), 6.7-6.9 (3H, m), 7.1-7.35 (5H, m).

### [Example 9]

### (2R,4R)-2-[2-[2-(2-(3,4-Difluorophenyl)ethyl]-4-fluorophenoxy]ethyl]-4-hydroxy-1-methylpyrrolidine hydrochloride (Exemplified Compound No. 1-92)

### (a) (2R,4R)-1-t-Butoxycarbonyl-4-t-butyldimethylsilyloxy-2-[2-[2-[2-(3,4-difluorophenyl)ethyl]-4-fluorophenoxy]ethyl]pyrrolidine

2-[2-(3,4-Difluorophenyl)ethyl]-4-fluorophenol (400 mg) was reacted with potassium t-butoxide (208 mg) and (2S,4R)-2-(2-bromoethyl)-1-t-butoxycarbonyl-4-t-butyldimethylsilyloxypyrrolidine (690 mg) in N,N-dimethylacetamide (5 ml) and treated in a similar manner to that described in the step (a) of Preparation Example 5. The oil thus obtained was purified by column chromatography on silica gel (eluent: hexane / ethyl acetate = 5/1), to give 580 mg (yield: 63%) of the title compound as a colorless oil.
NMR spectrum (270 MHz, CDCl₃) δ ppm: 0.02 (3H, s), 0.04 (3H, s), 0.84 (9H, s), 1.45 (9H, s), 1.7-1.95 (2H, m), 1.95-2.15 (1H, m), 2.2-2.55 (1H, m), 2.7-3.0 (4H, m), 3.25-3.65 (2H, m), 3.85-4.05 (2H, m), 4.05-4.25 (1H, m), 4.25-4.4 (1H, m), 6.7-7.1 (6H, m).

### (b) (2R,4R)-1-t-Butoxycarbonyl-2-[2-[2-[2-(3,4-difluorophenyl)ethyl]-4-fluorophenoxy]ethyl]-4-hydroxypyrrolidine

To a solution of (2R,4R)-1-t-butoxycarbonyl-4-t-butyldimethylsilyloxy-2-[2-[2-[2-(3,4-difluorophenyl)ethyl]-4-fluorophenoxy]ethyl)pyrrolidine (580 mg) from step (a) in tetrahydrofuran (5 ml) was added tetrabutylammonium fluoride (0.31 ml), followed by stirring at room temperature for one hour. The reaction mixture was concentrated by evaporation under reduced pressure, to afford oil. The oil was purified by column chromatography on silica gel (eluent: hexane / ethyl acetate = 1/1), to give 280 mg (yield: 61%) of the title compound as a colorless solid.
NMR spectrum (270 MHz, CDCl₃) δ ppm: 1.46 (9H, s), 1.7-2.0 (2H, m), 2.05-2.3 (1H, m), 2.3-2.55 (1H, m), 2.84 (4H, s), 3.4-3.7 (1H, m), 3.43 (1H, dd, J=4.2 and 11.9Hz), 3.85-4.05 (2H, m), 4.05-4.25 (1H, m), 4.35-4.5 (1H, m), 6.7-7.1 (6H, m).

### (c) (2R,4R)-2-[2-[2-[2-(3,4-Difluorophenyl)ethyl]-4-fluorophenoxy]ethyl]-4-hydroxy-1-methylpyrrolidine

(2R,4R)-1-t-Butoxycarbonyl-2-[2-[2-[2-(3,4-difluorophenyl)ethyl]-4-fluorophenoxy]ethyl]-4-hydroxypyrrolidine (280 mg) from step (b) was reacted with lithium aluminum hydride (50 mg ) in tetrahydrofuran (5 ml) and treated in a similar manner to that described in the step (b) of Preparation Example 1. The residue thus obtained was purified by column chromatography on silica gel (eluent: methylene chloride / methanol = 10/1), to give 140 mg (yield: 63%) of the title compound as a colorless solid.
NMR spectrum (270 MHz, CDCl₃) δ ppm: 1.75-2.5 (4H, m), 2.41 (1H, dd, J=4.3 and 10.8Hz), 2.51 (3H, s), 2.8-3.05 (1H, m), 2.84 (4H, s), 3.64 (1H, dd, J=6.0 and 10.8Hz), 3.85-4.1 (2H, m), 4.4-4.55 (1H, m), 6.8-6.9 (4H, m), 6.9-7.1 (2H, m).

### (d) (2R,4R)-2-[2-[2-[2-(3,4-Difluorophenyl)ethyl]-4-fluorophenoxy]ethyl]-4-hydroxy-1-methylpyrrolidine hydrochloride

To a solution of (2R,4R)-2-[2-[2-[2-(3,4-difluorophenyl)ethyl]-4-fluorophenoxy]ethyl]-4-hydroxy-1-methylpyrrolidine (140 mg) from step (c) in ethyl acetate (5 ml) was added a 4N hydrogen chloride - ethyl acetate solution (0.15 ml), to afford crystals. The crystals were collected by filtration and dried in vacuo, to give 113 mg (yield: 73%) of the title compound as colorless crystals.
Melting point: 93 - 94°C
NMR spectrum (400 MHz, DMSO + D₂O) δ ppm: 2.05-2.25 (1H, m), 2.25-2.7 (3H, m), 2.83 (4H, s), 2.9-3.15 (1H, m), 2.91 (3H, s), 3.75-4.3 (4H, m), 4.55-4.75 (1H, m), 6.7-7.15 (6H, m).

### [Example 10]

### (2R,4R)-2-[2-[2-[2-(3,4-Difluorophenyl)ethyl]-4-fluorophenoxy]ethyl]-4-hvdroxypyrrolidine hydrochloride (Exemplified Compound No. 1-133)

To a solution of (2R,4R)-1-t-butoxycarbonyl-2-[2-[2-[2-(3,4-difluorophenyl)ethyl]-4-fluorophenoxy]ethyl]-4-hydroxypyrrolidine (83 mg) from Preparation Example 9(b) in dioxane (2 ml) was added a 4N hydrogen chloride - dioxane solution (2 ml). The resulting mixture was allowed to stand at room temperature for one hour to afford crystals. The crystals were collected by filtration and dried in vacuo, to give 55 mg (yield: 77%) of the title compound as colorless crystals.
Melting point: 170 - 171°C
NMR spectrum (270 MHz, CD₃OD) δ ppm: 1.75-1.95 (1H, m), 2.15-2.35 (2H, m), 2.35-2.55 (1H, m), 2.85 (4H, s), 3.24 (1H, d, J=12.6Hz), 3.49 (1H, dd, J=4.4 and 12.6Hz), 3.95-4.2 (3H, m), 4.5-4.6 (1H, m), 6.7-7.15 (6H, m).

### [Example 11]

### (2R,4R)-2-[2-[6-fluoro-2-(2-phenylethyl)phenoxy]ethyl]-4-hydroxy-1-methylpyrrolidine hydrochloride (Exemplified Compound No. 1-136)

### (a) (2R,4R)-4-Dimethylcarbamoyloxy-2-[2-[6-fluoro-2-(2-phenylethyl)phenoxy]ethyl]-1-octyloxycarbonylpyrrolidine

6-Fluoro-2-(2-phenylethyl)phenol (520 mg) was reacted with potassium t-butoxide (300 mg) and (2S,4R)-2-(2-chloroethyl)-4-dimethylcarbamoyloxy-1-octyloxycarbonylpyrrolidine (820 mg) in N,N-dimethylacetamide (10 ml) and treated in a similar manner to that described in the step (a) of Preparation Example 5. The oil thus obtained was purified by column chromatography on silica gel (eluent: hexane / ethyl acetate = 2/1), to give 984 mg (yield: 81%) of the title compound as an oil.
NMR spectrum (270 MHz, CDCl₃) δ ppm: 0.8-0.95 (3H, m), 1.15-1.45 (10H, m), 1.55-1.7 (1H, m), 1.7-2.0 (1H, m), 2.0-2.15 (1H, m), 2.25-2.6 (2H, m), 2.75-3.0 (4H, m), 2.89 (6H, s), 3.54 (1H, dd, J=4.3 and 12.5Hz), 3.6-3.9 (1H, m), 3.95-4.25 (5H, m), 5.1-5.3 (1H, m), 6.8-7.0 (3H, m),7.1-7.3 (5H, m).

### (b) (2R,4R)-2-[2-[6-Fluoro-2-(2-phenylethyl)phenoxy]ethyl]-4-hydroxy-1-methylpyrrolidine

(2R,4R)-4-Dimethylcarbamoyloxy-2-[2-[6-fluoro-2-(2-phenylethyl)phenoxy]ethyl]-1-octyloxycarbonylpyrrolidine (984 mg) from step (a) was reacted with lithium aluminum hydride (200 mg) in tetrahydrofuran (20 ml) and treated in a similar manner to that described in the step (b) of Preparation Example 1. The residue thus obtained was purified by column chromatography on silica gel (eluent: methylene chloride / methanol = 5/1), to give 319 mg (yield: 53%) of the title compound as an oil.
NMR spectrum (270 MHz, CDCl₃) δ ppm: 1.55-1.75 (1H, m), 1.8-2.0 (2H, m), 2.15-2.35 (1H, m), 2.19 (1H, dd, J=5.4 and 10.1Hz), 2.37 (3H, s), 2.6-2.75 (1H, m), 2.8-3.0 (4H, m), 3.45 (1H, dd, J=6.3 and 10.1Hz), 3.95-4.15 (2H, m), 4.35-4.45 (1H, m), 6.85-7.0 (3H, m), 7.15-7.35 (5H, m).

### (c) (2R,4R)-2-[2-[6-Fluoro-2-(2-phenylethyl)phenoxy]ethyl]-4-hydroxy-1-methylpyrrolidine hydrochloride

To a solution of (2R,4R)-2-[2-[6-fluoro-2-(2-phenylethyl)phenoxy]ethyl]-4-hydroxy-1-methylpyrrolidine (319 mg) from step (b) in ethyl acetate (10 ml) was added a 4N hydrogen chloride - ethyl acetate solution (0.23 ml). The solvent was then distilled off under reduced pressure. The resulting oil was recrystallized from ethyl acetate. The crystals were collected by filtration and dried in vacuo, to give 320 mg (yield: 91%) of the title compound as colorless crystals.
Melting point: 136 - 138°C
NMR spectrum (270 MHz, CDCl₃) δ ppm: 2.0-2.2 (1H, m), 2.2-2.6 (3H, m), 2.8-3.1 (5H, m), 2.92 (3H, s), 3.8-4.25 (4H, m), 4.55-4.7 (1H, m), 6.85-7.05 (3H, m), 7.1-7.4 (5H, m).

### [Example 12]

### 2-[2-[4-Fluoro-2-(2-phenylethyl)phenoxy]ethyl]-1-methylpyrrolidine hydrochloride (Exemplified Compound No. 1-134)

### (a) 2-[-2-[4-Fluoro-2-(2-phenylethyl)phenoxylethyl]-1-methylpyrrolidine

To a solution of 4-fluoro-2-(2-phenylethyl)phenol (175 mg) in N,N-dimethylacetamide (5 ml) was added potassium t-butoxide (130 mg) with stirring in an ice-bath. To the mixture, 2-(2-chloroethyl)-1-methylpyrrolidine hydrochloride (220 mg) was added and the resulting mixture was stirred at room temperature for 5 hours and then at 55°C for 6 hours. The reaction mixture was cooled, diluted with ethyl acetate and then washed successively with water and brine. The ethyl acetate layer was dried and concentrated by evaporation under reduced pressure, to afford oil. The oil was purified by column chromatography on silica gel (eluent: methylene chloride / methanol = 5/1), to give 83.8 mg (yield: 32%) as an oil.
NMR spectrum (270 MHz, CDCl₃) δ ppm: 1.5-1.9 (4H, m), 1.9-2.1 (1H, m), 2.1-2.7 (3H, m), 2.36 (3H, s), 2.8-2.95 (4H, m), 3.05-3.15 (1H, m), 3.85-4.1 (2H, m), 6.7-6.9 (3H, m), 7.1-7.35 (5H, m).

### (b) 2-[2-[4-Fluoro-2-(2-phenylethyl)phenoxy]ethyl]-1-methylpyrrolidine hydrochloride

To a solution of the 2-[2-[4-fluoro-2-(2-phenylethyl)phenoxy]ethyl]-1-methylpyrrolidine (83.8 mg) from step (a) in a small amount of ethyl acetate was added a 4N hydrogen chloride - ethyl acetate solution (0.06 ml), to afford crystals. The crystals were collected by filtration and dried in vacuo, to give 68.9 mg (yield: 74%) of the title compound as colorless crystals.
Melting point: 149 - 151°C
NMR spectrum (270 MHz, CDCl₃) δ ppm: 1.9-2.15 (2H, m), 2.15-2.35 (2H, m), 2.35-2.6 (2H, m), 2.7-3.0 (5H, m), 2.74 (3H, s), 3.1-3.25 (1H, m), 3.8-4.05 (2H, m), 4.1-4.25 (1H, m), 6.7-6.95 (3H, m), 7.05-7.35 (5H, m).

### [Example 13]

### 3-[4-Fluoro-2-(2-phenylethyl)phenoxy]methyl-1-methylpiperidine hydrochloride (Exemplified Compound No. 1-135)

### (a) 1-t-Butoxycarbonyl-3-[4-fluoro-2-(2-phenylethyl)phenoxy]methylpiperidine

To a solution of 4-fluoro-2-(2-phenylethyl)phenol (175 mg) in N,N-dimethylacetamide (5 ml) was added potassium t-butoxide (100 mg) in an ice-bath, followed by stirring for 10 minutes. To the mixture, 1-t-butoxycarbonyl-3-tosyloxymethylpiperidine (330 mg) was added. The resulting mixture was then stirred at room temperature for 3 days. The reaction mixture was diluted with ethyl acetate and washed successively with water and brine. The ethyl acetate layer was dried and concentrated by evaporation under reduced pressure to obtain oil. The oil was purified by column chromatography on silica gel (eluent: hexane / ethyl acetate = 4/1), to give 291 mg (yield: 87%) of the title compound as an oil.
NMR spectrum (270 MHz, CDCl₃) δ ppm: 1.3-1.8 (3H, m), 1.43 (9H, s), 1.8-2.1 (2H, m), 2.7-3.1 (6H, m), 3.78 (2H, d, J=6.0Hz), 3.85-4.0 (1H, m), 4.0-4.2 (1H, m), 6.65-6.9 (3H, m), 7.1-7.35 (5H, m).

### (b) 3-[4-Fluoro-2-(2-phenylethyl)phenoxy]methyl-1-methylpiperidine

1-t-Butoxycarbonyl-3-[4-fluoro-2-(2-phenylethyl)phenoxy]methylpiperidine (291 mg) from step (a) was reacted with lithium aluminum hydride (100 mg) in tetrahydrofuran (10 ml) and treated in a similar manner to the step (b) of Preparation Example 1. The residue thus obtained was purified by column chromatography on silica gel (eluent: methylene chloride / methanol = 10/1), to give 89.8 mg (yield: 39%) of the title compound as an oil.
NMR spectrum (270 MHz, CDCl₃) δ ppm: 1.05-1.3 (1H, m), 1.6-2.05 (5H, m), 2.1-2.3 (1H, m), 2.30 (3H, s), 2.6-2.95 (5H, m), 2.95-3.05 (1H, m), 3.7-3.9 (2H, m), 6.7-6.9 (3H, m), 7.15-7.35 (5H, m).

### (c) 3-[4-Fluoro-2-(2-phenylethyl)phenoxy]methyl-1-methylpiperidine hydrochloride

To a solution of the 3-[4-fluoro-2-(2-phenylethyl)phenoxy]methyl-1-methylpiperidine (89.8 mg) from step (b) in a small amount of ethyl acetate was added a 4N hydrogen chloride - ethyl acetate solution (0.07 ml), to afford crystals. The crystals were collected by filtration and then dried in vacuo, to give 81.7 mg (yield: 82%) of the title compound as colorless crystals.
Melting point: 193 - 196°C
NMR spectrum (270 MHz, CDCl₃) δ ppm: 1.3-1.7 (1H, m), 1.85-2.05 (2H, m), 2.2-2.9 (8H, m), 2.72 (3H, s), 3.35-3.6 (2H, m), 3.7-4.0 (2H, m), 6.65-6.8 (1H, m), 6.8-6.95 (2H, m), 7.1-7.35 (5H, m).

### [Example 14]

### (2R,4R)-2-[2-[4-Fluoro-2-[2-(4-fluoro-3-methoxyphenyl)ethyl]phenoxy]ethyl]-4-lauroyloxy-1-methylpyrrolidine hydrochloride (Exemplified Compound No. 1-41)

### (a) (2R,4R)-2-[2-[4-Fluoro-2-[2-(4-fluoro-3-methoxyphenyl)ethyl]phenoxy]ethyl]-4-lauroyloxy-1-methylpyrrolidine

To a pyridine (10 ml) solution of (2R,4R)-2-[2-[4-fluoro-2-[2-(4-fluoro-3-methoxyphenyl)ethyl]phenoxy]ethyl]-4-hydroxy-1-methylpyrrolidine (513 mg) from the step (b) of Preparation Example 2 was added lauric anhydride (652 mg) and 4-dimethylaminopyridine (48 mg) at room temperature. The mixture was stirred at room temperature for 30 minutes and then at 40°C for one hour. The reaction mixture was diluted with ethyl acetate, washed twice with 1N-hydrochloric acid and then with brine. The ethyl acetate layer was dried and concentrated by evaporation under reduced pressure. The resulting oil was purified by column chromatography on silica gel (eluent: methylene chloride / methanol = 5/1), to give 684 mg (yield: 91%) of the title compound as colorless oil.
NMR spectrum (270 MHz, CDCl₃) δ ppm: 0.88 (3H, t, J=6.6Hz), 1.15-1.4 (16H, m), 1.45-1.85 (3H, m), 1.85-2.1 (2H, m), 2.15-2.3 (2H, m), 2.22 (2H, t, J=7.6Hz), 2.38 (3H, s), 2.55-2.7 (1H, m), 2.7-3.0 (4H, m), 3.60 (1H, dd, J=6.6 and 10.7Hz), 3.83 (3H, s), 3.85-4.05 (2H, m), 5.05-5.2 (1H, m), 6.6-7.05 (6H, m).

### (b) (2R,4R)-2-[2-[4-Fluoro-2-[2-(4-fluoro-3-methoxyphenyl)ethyl]phenoxy]ethyl]-4-lauroyloxy-1-methylpyrrolidine hydrochloride

To a solution of the (2R,4R)-2-[2-[4-fluoro-2-[2-(4-fluoro-3-methoxyphenyl)ethyl]phenoxy]ethyl]-4-lauroyloxy-1-methylpyrrolidine (684 mg) from step (a) in 10 ml of dioxane was added a 4N hydrogen chloride - dioxane solution (0.45 ml). The mixture was concentrated by evaporation under reduced pressure. Hexane was added to the residue to afford crystals. The crystals were collected by filtration and then dried in vacuo, to give 485 mg (yield: 67%) of the title compound as colorless crystals.
Melting point: 49 - 53°C
NMR spectrum (270 MHz, CDCl₃) δ ppm: 0.88 (3H, t, J=6.6Hz), 1.1-1.4 (16H, m), 1.4-1.7 (2H, m), 2.21 (2H, t, J=7.6Hz), 2.3-2.5 (2H, m), 2.5-2.7 (2H, m), 2.75-3.0 (5H, m), 2.86 (3H, s), 3.45-3.7 (1H, m), 3.83 (3H, s), 3.9-4.05 (1H, m), 4.1-4.25 (1H, m), 4.25-4.45 (1H, m), 5.3-5.4 (1H, m), 6.55-7.05 (6H, m).

### [Example 15]

### (2R,4R)-2-[2-[4-Chloro-2-(2-phenylethyl)phenoxy]ethyl]-4-hydroxy-1-methylpyrrolidine (Exemplified Compound No. 1-137)

### (a) (2R,4R)-2-[2-[4-Chloro-2-(2-phenylethyl)phenoxy]ethyl]-1-ethoxycarbonyl-4-hydroxypyrrolidine

4-Chloro-2-(2-phenylethyl)phenol (500 mg) was reacted with potassium t-butoxide (270 mg) and (2S,4R)-2-(2-chloroethyl)-1-ethoxycarbonyl-4-hydroxypyrrolidine (520 mg) in N,N-dimethylacetamide (10 ml) and treated in a similar manner to that described in the step (a) of Preparation Example 1. The oil thus obtained was purified by column chromatography on silica gel (eluent: hexane / ethyl acetate = 1/2), to give 260 mg of the title compound (yield: 29%) as an oil.
NMR spectrum (270 MHz, CDCl₃) δ ppm: 1.1-1.35 (3H, m), 1.75-2.0 (2H, m), 2.05-2.55 (2H, m), 2.85 (4H, s), 3.4-3.75 (1H, m), 3.41 (1H, dd, J=4.2 and 11.9Hz), 3.9-4.3 (5H, m), 4.3-4.45 (1H, m), 6.73 (1H, d, J=8.6Hz), 7.05-7.35 (7H, m).

### (b) (2R,4R)-2-[2-[4-Chloro-2-(2-phenylethyl)phenoxylethyl]-4-hydroxy-1-methylpyrrolidine

(2R,4R)-2-[2-[4-Chloro-2-(2-phenylethyl)phenoxy]ethyl]-1-ethoxycarbonyl-4-hydroxypyrrolidine (260 mg) from step (a) was reacted with lithium aluminum hydride (70 mg) in tetrahydrofuran (10 ml) and treated in a similar manner to that described in the step (b) of Preparation Example 1. The residue thus obtained was purified by column chromatography on silica gel (eluent: methylene chloride / methanol = 5/1), to give 103 mg (yield: 46%) of the title compound as colorless solid.
Melting point: 65 - 68°C
NMR spectrum (270 MHz, CDCl₃) δ ppm: 1.7-2.05 (3H, m), 2.15-2.4 (1H, m), 2.31 (1H, dd, J=4.9 and 10.5Hz), 2.44 (3H, s), 2.75-2.95 (1H, m), 2.86 (4H, s), 3.55(1H, dd, J=6.1 and 10.5Hz), 3.85-4.1 (2H, m), 4.35-4.5 (1H, m), 6.74 (1H, d, J=8.4Hz), 7.05-7.35 (7H, m).

### [Example 16]

### (2R,4R)-2-[2-[4-Bromo-2-(2-phenylethyl)phenoxy]ethyl]-4-hydroxy-1-methylpyrrolidine (Exemplified Compound No. 1-138)

### (a) (2R,4R)-2-[2-[4-Bromo-2-(2-phenylethyl)phenoxy]ethyl]-1-ethoxycarbonyl-4-hydroxypyrrolidine

4-bromo-2-(2-phenylethyl)phenol (500 mg) was reacted with potassium t-butoxide (220 mg) and (2S,4R)-2-(2-chloroethyl)-1-ethoxycarbonyl-4-hydroxypyrrolidine (440 mg) in N,N-dimethylacetamide (10 ml) and treated in a similar manner to that described in the step (a) of Preparation Example 1. The oil thus obtained was purified by column chromatography on silica gel (eluent: hexane / ethyl acetate = 1/2), to give 280 mg (yield: 34%) of the title compound as an oil.
NMR spectrum (270 MHz, CDCl₃) δ ppm: 1.1-1.35 (3H, m), 1.75-2.6 (4H, m), 2.85 (4H, s), 3.4-3.75 (1H, m), 3.42 (1H, dd, J=4.2 and 11.9Hz), 3.9-4.3 (5H, m), 4.3-4.45 (1H, m), 6.69(1H, d, J=8.5Hz), 7.15-7.35 (7H, m).

### (b) (2R,4R)-2-[2-[4-Bromo-2-(2-phenylethyl)phenoxy]ethyl]-4-hydroxy-1-methylpyrrolidine

(2R,4R)-2-[2-[4-Bromo-2-(2-phenylethyl)phenoxy]ethyl]-1-ethoxycarbonyl-4-hydroxypyrrolidine (280 mg) form step (a) was reacted with lithium aluminum hydride (70 mg) in tetrahydrofuran (10 ml) and treated in a similar manner to that described in the step (b) of Preparation Example 1. The residue thus obtained was purified by column chromatography on silica gel (eluent: methylene chloride / methanol = 5/1), to give 113 mg (yield: 46%) of the title compound as colorless solid.
Melting point: 63 - 66°C
NMR spectrum (270 MHz, CDCl₃) δ ppm: 1.7-2.05 (3H, m), 2.1-2.35 (1H, m), 2.29 (1H, dd, J=4.9 and 10.4Hz), 2.42 (3H, s), 2.7-2.95 (1H, m), 2.86 (4H, s), 3.52 (1H, dd, J=6.1 and 10.4Hz), 3.9-4.05 (2H, m), 4.35-4.5 (1H, m), 6.70 (1H, d J=8.4Hz), 7.15-7.35 (7H, m).

### [Example 17]

### (2R,4R)-2-[2-[5-Chloro-2-(2-phenylethyl)phenoxy]ethyl]-4-hydroxy-1-methylpyrrolidine hydrochloride (Exemplified Compound No. 1-140)

### (a) (2R,4R)-2-[2-[5-Chloro-2-(2-phenylethyl)phenoxy]ethyl]-4-dimethylcarbamoyloxy-1-octyloxycarbonylpyrrolidine

To a solution of 5-chloro-2-(2-phenylethyl)phenol (680 mg) in N,N-dimethylacetamide (10 ml) was added potassium t-butoxide (360 mg) in an ice-bath, followed by stirring for 10 minutes. To the mixture, (2S,4R)-2-(2-chloroethyl)-4-dimethylcarbamoyloxy-1-octyloxycarbonylpyrrolidine (1000 mg) was added. The resulting mixture was treated in a similar manner to that described in the step (a) of Preparation Example 5, to afford oil. The resulting oil was purified by column chromatography on silica gel (eluent: hexane / ethyl acetate = 1/1), to give 1.38 g (yield: 91%) of the title compound as an oil.
NMR spectrum (270 MHz, CDCl₃) δ ppm: 0.8-1.0 (3H, m), 1.15-1.45 (10H, m), 1.45-1.7 (2H, m), 1.75-2.15 (2H, m), 2.25-2.7 (2H, m), 2.7-3.0 (4H, m), 2.85 (3H, s), 2.87 (3H, s), 3.53 (1H, dd, J=4.1 and 12.6Hz), 3.6-3.9 (1H, m), 3.9-4.3 (5H, m), 5.1-5.3 (1H, m), 6.7-6.9 (2H, m), 6.97 (1H, d, J=7.9Hz), 7.1-7.35 (5H, m).

### (b) (2R,4R)-2-[2-[5-Chloro-2-(2-phenylethyl)phenoxylethyl]-4-hydroxy-1-methylpyrrolidine

(2R,4R)-2-[2-[5-Chloro-2-(2-phenylethyl)phenoxy]ethyl)-4-dimethylcarbamoyloxy-1-octyloxycarbonylpyrrolidine (1380 mg) from step (a) was reacted with lithium aluminum hydride (450 mg) in tetrahydrofuran (20 ml) and treated in a similar manner to that described in the step (b) of Preparation Example 1. The residue thus obtained was purified by column chromatography on silica gel (eluent: methylene / methanol = 5/1), to give 256 mg (yield: 30%) of the title compound as colorless solid.
NMR spectrum (270 MHz, CDCl₃) δ ppm: 1.6-1.8 (1H, m), 1.8-2.05 (2H, m), 2.1-2.3 (1H, m), 2.22 (1H, dd J=5.4 and 10.1Hz), 2.39 (3H, s), 2.6-2.75 (1H, m), 2.8-2.95 (4H, m), 3.48 (1H, dd, J=6.3 and 10.1Hz), 3.9-4.1 (2H, m), 4.35-4.5 (1H, m), 6.75-6.9 (2H, m), 6.99 (1H, d J=7.8Hz), 7.1-7.35 (5H, m).

### (c) (2R,4R)-2-[2-[5-Chloro-2-(2-phenylethyl)phenoxy]ethyl]-4-hydroxy-1-methylpyrrolidine hydrochloride

To a solution of (2R,4R)-2-[2-[5-chloro-2-(2-phenylethyl)phenoxy]ethyl]-4-hydroxy-1-methylpyrrolidine (256 mg) from step (b) in ethyl acetate (5 ml) was added a 4N hydrogen chloride - ethyl acetate solution (0.18 ml). The solvent was then distilled off under reduced pressure. The oil thus obtained was dissolved in 10 ml of ethyl acetate. The resulting solution was allowed to stand to afford crystals. The crystals were collected by filtration and dried in vacuo, to give 183 mg (yield: 65%) of the title compound as colorless crystals.
Melting point: 99 - 102°C
NMR spectrum (270 MHz, CDCl₃) δ ppm: 2.05-2.25 (1H, m), 2.31 (1H, dd, J=5.9 and 13.8Hz), 2.35-2.65 (2H, m), 2.8-3.0 (5H, m), 2.86 (3H, s), 3.7-3.9 (1H, m), 3.9-4.25 (3H, m), 4.55-4.7 (1H, m), 6.82 (1H, d, J=1.9Hz), 6.85-7.0 (1H, m), 7.02 (1H, d, J=8.0Hz), 7.1-7.35 (5H, m).

### [Example 18]

### (2R,4R)-4-Hydroxy-2-[2-[2-[2-(3-methoxyphenyl)ethyl]phenoxy]ethyl]-1-methylpyrrolidine hydrochloride (Exemplified Compound No. 1-13)

### (a) (2R,4R)-4-Benzyloxy-1-ethoxycarbonyl-2-[2-[2-[2-(3-methoxyphenyl)ethyl]phenoxy]ethyl]pyrrolidine

To a solution of 2-[2-(3-methoxyphenyl)ethyl]phenol (500 mg) in N,N-dimethylacetamide (20 ml) was added potassium t-butoxide (270 mg) in an ice-bath, followed by stirring for 15 minutes. To the reaction mixture, (2S,4R)-4-benzyloxy-1-ethoxycarbonyl-2-[2-(p-toluenesulfonyloxy)ethyl]pyrrolidine (1190 mg) was added. The mixture was treated in a similar manner to that described in the step (a) of Preparation Example 5, to afford oil. The oil was purified by column chromatography on silica gel (eluent: hexane / ethyl acetate = 4/1), to give 860 mg (yield: 78%) of the title compound as an oil.
NMR spectrum (270 MHz, CDCl₃) δ ppm: 1.1-1.35 (3H, m), 1.75-2.1 (2H, m), 2.2-2.6 (2H, m), 2.8-3.0 (4H, m), 3.43 (1H, dd, J=4.6 and 11.9Hz), 3.55-4.3 (7H, m), 3.75 (3H, s), 4.45 (2H, s), 6.65-6.9 (5H, m), 7.05-7.4 (8H, m).

### (b) (2R,4R)-1-Ethoxycarbonyl-4-hydroxy-2-[2-[2-[2-(3-methoxyphenyl)ethyl]phenoxy]ethyl]pyrrolidine

A solution of (2R,4R)-4-benzyloxy-1-ethoxycarbonyl-2-[2-[2-[2-(3-methoxyphenyl)ethyl]phenoxy]ethyl]pyrrolidine (853 mg) from step (a) in ethanol (6 ml) was stirred in the presence of a 5% palladium-carbon catalyst (85 mg) under a hydrogen atmosphere at 60°C for 7 hours. The reaction mixture was cooled and the catalyst was filtered off. The filtrate was concentrated by evaporation under reduced pressure. The residue was purified by column chromatography on silica gel (eluent: hexane / ethyl acetate = 1/1), to give 650 mg (yield: 93%) of the title compound as colorless oil.
NMR spectrum (270 MHz, CDCl₃) δ ppm: 1.1-1.35 (3H, m), 1.7-2.3 (3H, m), 2.3-2.6 (1H, m), 2.8-3.0 (4H, m), 3.46 (1H, dd, J=4.6 and 11.9Hz). 3.5-3.9 (1H, m), 3.78 (3H, s), 3.95-4.3 (5H, m), 4.35-4.5 (1H, m), 6.7-6.95 (5H, m), 7.1-7.3 (3H, m).

### (c) (2R,4R)-4-Hydroxy-2-[2-[2-[2-(3-methoxyphenyl)ethyl]phenoxy]ethyl]-1-methylpyrrolidine

(2R,4R)-1-Ethoxycarbonyl-4-hydroxy-2-[2-[2-[2-(3-methoxyphenyl)ethyl]phenoxy]ethyl]pyrrolidine (640 mg) from step (b) was reacted with lithium aluminum hydride (176 mg) in tetrahydrofuran (30 ml) and treated in a similar manner to that described in the step (b) of Preparation Example 1. The residue thus obtained was purified by column chromatography on silica gel (eluent: methylene chloride / methanol = 10/1), to give 523 mg (yield: 95%) of the title compound as a colorless oil.
NMR spectrum (270 MHz, CDCl₃) δ ppm: 1.7-2.5 (5H, m), 2.48 (3H, s), 2.8-3.0 (5H, m), 3.59 (1H, dd, J=5.9 and 10.6Hz), 3.78 (3H, s), 3.9-4.2 (2H, m), 4.4-4.5 (1H, m), 6.7-7.0 (5H, m), 7.1-7.3 (3H, m).

### (d) (2R,4R)-4-Hydroxy-2-[2-[2-[2-(3-methoxyphenyl)ethyl]phenoxy]ethyl]-1-methylpyrrolidine hydrochloride

To a solution of (2R,4R)-4-hydroxy-2-[2-[2-[2-(3-methoxyphenyl)ethyl]phenoxy]ethyl]-1-methylpyrrolidine (520 mg) from step (c) in dioxane (5 ml) was added a 4N hydrogen chloride - dioxane solution (1.1 ml). The solvent was distilled off under reduced pressure. The oil thus obtained was dissolved in 2 ml of methylene chloride, followed by the addition of 40 ml of ethyl acetate. The resulting mixture was allowed to stand at room temperature to afford crystals. The crystals were collected by filtration and then dried in vacuo, to give 420 mg (yield: 73%) of the title compound as colorless crystals.
Melting point: 100 - 102°C
NMR spectrum (400 MHz, CDCl₃ + D₂O) δ ppm: 2.0-2.2 (1H, m), 2.3-2.65 (3H, m), 2.75-3.1 (5H, m), 2.88 (3H, s), 3.77 (3H, s), 3.8-4.3 (4H, m), 4.55-4.7 (1H, m), 6.7-6.8 (3H, m), 6.83 (1H, d, J=8.1Hz), 6.92 (1H, t, J=7.3Hz), 7.1-7.3 (3H, m).

### [Formulation Example 1]

### Capsule

| | |
|---|---|
| Compound of Example 3 | 20.0 mg |
| Lactose | 158.7 |
| Corn starch | 70.0 |
| Magnesium stearate | 1.3 |
| | 250 mg |

The powders of the compounds listed above were mixed and passed through a 60-mesh sieve and then filled in a No.3 capsule for 250 mg, to give a capsule.

### [Formulation Example 2]

### Tablet

| | |
|---|---|
| Compound of Example 3 | 20.0 mg |
| Lactose | 154.0 |
| Corn starch | 25.0 |
| Magnesium stearate | 1.0 |
| | 200 mg |

The powders of the compounds listed above were mixed and tableted by a tablet machine, to give a tablet (200 mg).

The tablet can be coated with sugar, if necessary.

### [Industrial Applicability]

The compound of the formula (I) has excellent pancreatitis inhibitory activity and low toxicity. It, therefore, is useful as a theraputic or preventive (preferably therapeutic) agent for pancreatitis. The compound wherein R⁴ represents an acyloxy-heterocyclic group (particularly, aliphatic acyloxy-pyrrolidinyl group) is characterized by the property of causing less irritation to the stomach.

For the use as a therapeutic or preventive agent for pancreatitis, the compound (I) of the present invention or a pharmacologically acceptable salt thereof can be administered orally in the form of tablets, capsules, granules, powders, syrups and the like or non-orally in the form of injections and the like, without or with mixing with a suitable pharmacologically acceptable excipient, diluent and the like.

The above formulations can be prepared using additives by methods familiar to those skilled in the art. Examples of the additives may be, for example, excipients (for example, sugar derivatives such as lactose, sucrose, glucose, mannitol or sorbitol; starch derivatives such as corn starch, potato starch, α-starch, dextrin or carboxymethyl starch; cellulose derivatives such as crystalline cellulose, low-substituted hydroxypropylcellulose, hydroxypropylmethylcellulose, carmellose, carmellose calcium or internally-crosslinked carmellose sodium carboxymethylcellulose; acacia; dextran; pullulan; silicate derivatives such as light anhydrous silicic acid, synthetic aluminum silicate or magnesium aluminometasilicate; phosphate derivative such as calcium hydrogen phosphate; carbonate derivatives such as calcium carbonate; or sulfate derivatives such as calcium sulfate), binders (for example, the above-exemplified excipients, gelatin, polyvinylpyrrolidone; or Macrogol), decay agents (for example, the above-exemplified excipients or chemically modified starch or cellulose derivatives such as croscarmellose sodium, sodium carboxymethyl starch or crosslinked polyvinylpyrrolidone), lubricants (for example, talc, stearic acid, metal salts of stearic acid such as calcium stearate or magnesium stearate; colloidal silica; waxes such as beeswax or spermaceti; boric acid; glycol; carboxylic acids such as fumaric acid or adipic acid; sodium carboxylates such as sodium benzoate; sulfates such as sodium sulfate; leucine; lauryl sulfates such as sodium lauryl sulfate or magnesium lauryl sulfate; silicic acids such as anhydrous silicic acid or silicic acid hydrate; or starch derivatives exemplified above as the excipient), stabilizers (for example, paraoxybenzoates such as methyl paraben or propyl paraben; alcohols such as chlorobutanol, benzyl alcohol or phenylethyl alcohol; benzalkonium chloride; phenol derivatives such as phenol or cresol; thimerosal; acetic anhydride; or sorbic acid), taste or odor - masking agents (for example, generally used sweetening agents, acidulants or flavors), diluents and solvents for injection (ex. water, ethanol and glycerin). Although the dose of the compound of the invention depends on the condition, age, administration route and the like, it is orally administered in an amount of 1 mg (preferably 10 mg) in a single dose as a lower limit and 1000 mg (preferably 500 mg) in a single dose as an upper limit, while it is intravenously administered in an amount of 0.5 mg (preferably 5 mg) in a single dose as a lower limit and 500 mg (preferably 250 mg) in a single dose as an upper limit. It is desired to be administered to an adult one to three times daily depending on the condition of the patient.

## Claims

1. A composition for the treatment or prevention of pancreatitis, which comprises as an active ingredient a diarylalkane derivative represented by the following formula: 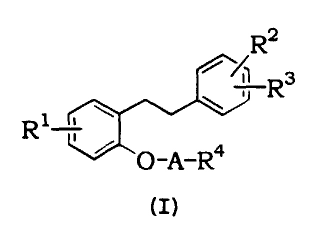 [wherein:
R¹ represents a hydrogen atom or a halogen atom,
R² and R³ are the same or different and each independently represents a hydrogen atom, a halogen atom or a C₁-C₄ alkoxy group,
R⁴ represents a substituted or unsubstituted 5- or 6-membered cyclic amino group which may further contain an oxygen or sulfur atom (said substituent on a carbon atom represents a hydroxy group or a C₁-C₂₀ aliphatic acyloxy group which may contain double bonds and said substituent on the nitrogen atom represents a C₁-C₄ alkyl group), and
A represents a C₁-C₄ alkylene group], or a pharmacologically acceptable salt thereof.

2. A composition according to claim 1, wherein an active ingredient is a diarylalkane derivative wherein R¹ represents a hydrogen, fluorine or chlorine atom, or a pharmacologically acceptable salt thereof.

3. A composition according to claim 1, wherein an active ingredient is a diarylalkane derivative wherein R¹ represents a hydrogen or fluorine atom, or a pharmacologically acceptable salt thereof.

4. A composition according to any one of claims 1 to 3, wherein an active ingredient is a diarylalkane derivative wherein R² and R³ are the same or different and each independently represents a hydrogen atom, fluorine atom, chlorine atom, methoxy group or ethoxy group, or a pharmacologically acceptable salt thereof.

5. A composition according to any one of claims 1 to 3, wherein an active ingredient is a diarylalkane derivative wherein R² represents a hydrogen atom, fluorine atom or chlorine atom and R³ represents a methoxy group, or a pharmacologically acceptable salt thereof.

6. A composition according to any one of claims 1 to 3, wherein an active ingredient is a diarylalkane derivative wherein R² represents a hydrogen atom or fluorine atom and R³ represents a methoxy group, or a pharmacologically acceptable salt thereof.

7. A composition according to any one of claims 1 to 6, wherein an active ingredient is a diarylalkane derivative wherein R⁴ represents a substituted or unsubstituted, pyrrolidinyl, piperidyl, morpholinyl or thiomorpholinyl group (said substituent on a carbon atom represents a hydroxy, C₂-C₅ aliphatic acyloxy, C₈-C₁₈ aliphatic acyloxy, acryloyl, crotonoyl, oleoyl or linoleoyl group, and said substituent on the nitrogen atom represents a methyl or ethyl group), or a pharmacologically acceptable salt thereof.

8. A composition according to any one of claims 1 to 6, wherein an active ingredient is a diarylalkane derivative wherein R⁴ represents a substituted or unsubstituted, pyrrolidinyl, piperidyl or morpholinyl group (said substituent on a carbon atom represents a hydroxy or C₈-C₁₈ aliphatic acyloxy group, and said substituent on the nitrogen atom represents a methyl group), or a pharmacologically acceptable salt thereof.

9. A composition according to any one of claims 1 to 6, wherein an active ingredient is a diarylalkane derivative wherein R⁴ represents a 2-pyrrolidinyl, 3-pyrrolidinyl, 1-methyl-2-pyrrolidinyl, 4-hydroxy-2-pyrrolidinyl, 4-octanoyloxy-2-pyrrolidinyl, 4-decanoyloxy-2-pyrrolidinyl, 4-lauroyloxy-2-pyrrolidinyl, 4-myristoyloxy-2-pyrrolidinyl, 4-palmitoyloxy-2-pyrrolidinyl, 4-stearoyloxy-2-pyrrolidinyl, 1-methyl-4-hydroxy-2-pyrrolidinyl, 1-methyl-4-octanoyloxy-2-pyrrolidinyl, 1-methyl-4-decanoyloxy-2-pyrrolidinyl, 1-methyl-4-lauroyloxy-2-pyrrolidinyl, 1-methyl-4-myristoyloxy-2-pyrrolidinyl, 1-methyl-4-palmitoyloxy-2-pyrrolidinyl, 1-methyl-4-stearoyloxy-2-pyrrolidinyl, 2-piperidyl, 3-piperidyl, 4-piperidyl, 1-methyl-2-piperidyl, 1-methyl-3-piperidyl, 1-methyl-4-piperidyl, 2-morpholinyl or 4-methyl-2-morpholinyl group; or a pharmacologically acceptable salt thereof.

10. A composition according to any one of claims 1 to 6, wherein an active ingredient is a diarylalkane derivative wherein R⁴ represents a 2-pyrrolidinyl, 1-methyl-2-pyrrolidinyl, 4-hydroxy-2-pyrrolidinyl, 4-decanoyloxy-2-pyrrolidinyl, 4-lauroyloxy-2-pyrrolidinyl, 1-methyl-4-hydroxy-2-pyrrolidinyl, 1-methyl-4-decanoyloxy-2-pyrrolidinyl or 1-methyl-4-lauroyloxy-2-pyrrolidinyl group; or a pharmacologically acceptable salt thereof.

11. A composition according to any one of claims 1 to 10, wherein an active ingredient is a diarylalkane derivative wherein A represents a C₁-C₃ alkylene group; or a pharmacologically acceptable salt thereof.

12. A composition according to any one of claims 1 to 10, wherein an active ingredient is a diarylalkane derivative wherein A represents a methylene or ethylene group; or a pharmacologically acceptable salt thereof.

13. A composition according to any one of claims 1 to 10, wherein an active ingredient is a diarylalkane derivative wherein A represents an ethylene group; or a pharmacologically acceptable salt thereof.

14. A composition according to claim 1, wherein an active ingredient is a diarylalkane derivative which is,
4-hydroxy-2-[2-[2-[2-(3-methoxyphenyl)ethyl]phenoxy]ethyl]-1-methylpyrrolidine,
2-[2-[4-fluoro-2-[2-(3-methoxyphenyl)ethyl]phenoxy]ethyl]-4-hydroxy-1-methylpyrrolidine,
2-[2-[2-[2-(4-fluoro-3-methoxyphenyl)ethyl]phenoxy)ethyl]-4-hydroxy-1-methylpyrrolidine,
2-[2-[4-fluoro-2-[2-(4-fluoro-3-methoxyphenyl)ethyl]phenoxy]ethyl]-4-hydroxy-1-methylpyrrolidine,
4-decanoyloxy-2-[2-[4-fluoro-2-[2-(3-methoxyphenyl)ethyl]phenoxy]ethyl]-1-methylpyrrolidine,
4-decanoyloxy-2-[2-[2-[2-(4-fluoro-3-methoxyphenyl)ethyl]phenoxy]ethyl]-1-methylpyrrolidine,
4-decanoyloxy-2-[2-[4-fluoro-2-[2-(4-fluoro-3-methoxyphenyl)ethyl]phenoxy]ethyl]-1-methylpyrrolidine,
4-lauroyloxy-2-[2-[2-[2-(3-methoxyphenyl)ethyl]phenoxy]ethyl]-1-methylpyrrolidine
2-[2-[4-fluoro-2-[2-(3-methoxyphenyl)ethyl]phenoxy)ethyl]-4-lauroyloxy-1-methylpyrrolidine,
2-[2-[2-(2-(4-fluoro-3-methoxyphenyl)ethyl]phenoxy]ethyl]-4-lauroyloxy-1-methylpyrrolidine, or
2-[2-[4-fluoro-2-[2-(4-fluoro-3-methoxyphenyl)ethyl]phenoxy]ethyl]-4-lauroyloxy-1-methylpyrrolidine; or a pharmacologically acceptable salt thereof.

15. A method for the treatment or prevention of pancreatitis, which comprises administering to a warm-blooded animal in need of such treatment and prevention an effective amount of a diarylalkane derivative represented by the following formula: 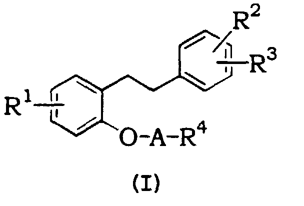 [wherein:
R¹ represents a hydrogen atom or a halogen atom,
R² and R³ are the same or different and each independently represents a hydrogen atom, a halogen atom or a C₁-C₄ alkoxy group,
R⁴ represents a substituted or unsubstituted 5- or 6-membered cyclic amino group which may further contain an oxygen or sulfur atom (said substituent on a carbon atom represents a hydroxy group or a C₁-C₂₀ aliphatic acyloxy group which may contain double bonds and said substituent on the nitrogen atom represents a C₁-C₄ alkyl group), and
A represents a C₁-C₄ alkylene group], or a pharmacologically acceptable salt thereof.

16. A method for the treatment or prevention of pancreatitis according to claim 15, wherein in the formula (I), R¹ represents a hydrogen, fluorine or chlorine atom.

17. A method for the treatment or prevention of pancreatitis according to claim 15, wherein in the formula (I), R¹ represents a hydrogen or fluorine atom.

18. A method for the treatment or prevention of pancreatitis according to any one of claims 15 to 17, wherein in the formula (I), R² and R³ are the same or different and each independently represents a hydrogen atom, fluorine atom, chlorine atom, methoxy group or ethoxy group.

19. A method for the treatment or prevention of pancreatitis according to any one of claims 15 to 17, wherein in the formula (I), R² represents a hydrogen atom, fluorine atom or chlorine atom and R³ represents a methoxy group.

20. A method for the treatment or prevention of pancreatitis according to any one of claims 15 to 17, wherein in the formula (I), R² represents a hydrogen atom or fluorine atom and R³ represents a methoxy group.

21. A method for the treatment or prevention of pancreatitis according to any one of claims 15 to 20, wherein in the formula (I), R⁴ represents a substituted or unsubstituted, pyrrolidinyl, piperidyl, morpholinyl or thiomorpholinyl group (said substituent on a carbon atom represents a hydroxy, C₂-C₅ aliphatic acyloxy, C₈-C₁₈ aliphatic acyloxy, acryloyl, crotonoyl, oleoyl or linoleoyl group, and said substituent on the nitrogen atom represents a methyl or ethyl group).

22. A method for the treatment or prevention of pancreatitis according to any one of claims 15 to 20, wherein in the formula (I), R⁴ represents a substituted or unsubstituted, pyrrolidinyl, piperidyl or morpholinyl group (said substituent on a carbon atom represents a hydroxy or C₈-C₁₈ aliphatic acyloxy group and said substituent on the nitrogen atom represents a methyl group).

23. A method for the treatment or prevention of pancreatitis according to any one of claims 15 to 20, wherein in the formula (I), R⁴ represents a 2-pyrrolidinyl, 3-pyrrolidinyl, 1-methyl-2-pyrrolidinyl, 4-hydroxy-2-pyrrolidinyl, 4-octanoyloxy-2-pyrrolidinyl, 4-decanoyloxy-2-pyrrolidinyl, 4-lauroyloxy-2-pyrrolidinyl, 4-myristoyloxy-2-pyrrolidinyl, 4-palmitoyloxy-2-pyrrolidinyl, 4-stearoyloxy-2-pyrrolidinyl, 1-methyl-4-hydroxy-2-pyrrolidinyl, 1-methyl-4-octanoyloxy-2-pyrrolidinyl, 1-methyl-4-decanoyloxy-2-pyrrolidinyl, 1-methyl-4-lauroyloxy-2-pyrrolidinyl, 1-methyl-4-myristoyloxy-2-pyrrolidinyl, 1-methyl-4-palmitoyloxy-2-pyrrolidinyl, 1-methyl-4-stearoyloxy-2-pyrrolidinyl, 2-piperidyl, 3-piperidyl, 4-piperidyl, 1-methyl-2-piperidyl, 1-methyl-3-piperidyl, 1-methyl-4-piperidyl, 2-morpholinyl or 4-methyl-2-morpholinyl group.

24. A method for the treatment or prevention of pancreatitis according to any one of claims 15 to 20, wherein in the formula (I), R⁴ represents a 2-pyrrolidinyl, 1-methyl-2-pyrrolidinyl, 4-hydroxy-2-pyrrolidinyl, 4-decanoyloxy-2-pyrrolidinyl, 4-lauroyloxy-2-pyrrolidinyl, 1-methyl-4-hydroxy-2-pyrrolidinyl, 1-methyl-4-decanoyloxy-2-pyrrolidinyl or 1-methyl-4-lauroyloxy-2-pyrrolidinyl group.

25. A method for the treatment or prevention of pancreatitis according to any one of claims 15 to 24, wherein in the formula (I), A represents a C₁-C₃ alkylene group.

26. A method for the treatment or prevention of pancreatitis according to any one of claims 15 to 24, wherein in the formula (I), A represents a methylene or ethylene group.

27. A method for the treatment or prevention of pancreatitis according to any one of claims 15 to 24, wherein in the formula (I), A represents a ethylene group.

28. A method for the treatment or prevention of pancreatitis according to claim 15, wherein the compound of the formula (I) is:
4-hydroxy-2-[2-[2-[2-(3-methoxyphenyl)ethyl]phenoxy]ethyl]-1-methylpyrrolidine,
2-[2-[4-fluoro-2-[2-(3-methoxyphenyl)ethyl)phenoxy]ethyl]-4-hydroxy-1-methylpyrrolidine,
2-[2-[2-[2-(4-fluoro-3-methoxyphenyl)ethyl]phenoxy]ethyl]-4-hydroxy-1-methylpyrrolidine,
2-[2-[4-fluoro-2-[2-(4-fluoro-3-methoxyphenyl)ethyl]phenoxy]ethyl]-4-hydroxy-1-methylpyrrolidine,
4-decanoyloxy-2-[2-[4-fluoro-2-[2-(3-methoxyphenyl)ethyl]phenoxy]ethyl]-1-methylpyrrolidine,
4-decanoyloxy-2-[2-[2-[2-(4-fluoro-3-methoxyphenyl)ethyl]phenoxy)ethyl]-1-methylpyrrolidine,
4-decanoyloxy-2-[2-[4-fluoro-2-[2-(4-fluoro-3-methoxyphenyl)ethyl]phenoxy]ethyl]-1-methylpyrrolidine,
4-lauroyloxy-2-[2-[2-[2-(3-methoxyphenyL)ethyl]phenoxy]ethyl]-1-methylpyrrolidine
2-[2-[4-fluoro-2-[2-(3-methoxyphenyl)ethyl]phenoxy]ethyl]-4-lauroyloxy-1-methylpyrrolidine,
2-[2-[2-[2-(4-fluoro-3-methoxyphenyl)ethyl]phenoxy]ethyl]-4-lauroyloxy-1-methylpyrrolidine, or
2-[2-[4-fluoro-2-[2-(4-fluoro-3-methoxyphenyl)ethyl]phenoxy]ethyl]-4-lauroyloxy-1-methylpyrrolidine.
